# EUROPEAN PATENT APPLICATION

(11) **EP 1 059 533 A1**
(43) Date of publication of application: **13.12.2000**
(21) Application number: 99906489.2
(22) Date of filing: 25.02.1999
(51) Int. Cl.: G01N 33/577, C07K 14/47, C07K 19/00, C12N 15/12, C07K 16/18

(54) **METHOD FOR IMMUNOCHEMICALLY ASSAYING ANTI-HM1.24 ANTIBODY**

(30) Priority: 25.02.1998 JP 6061398
(71) Applicant: CHUGAI SEIYAKU KABUSHIKI KAISHA, Tokyo, 115-8543 (JP)
(72) Inventor: OZAKI, Yasuko Chugai Seiyaku Kabushiki Kaisha, Shizuoka 412-8513 (JP); KOISHIHARA, Yasuo Chugai Seiyaku Kabushiki Kaisha, Shizuoka 412-8513 (JP)
(74) Representative: Harding, Charles Thomas
(86) International application number: JP9900885
(87) International publication number: WO9943703

(57) **Abstract**

An immunochemical assay for anti-HM1.24 antibody, said method comprising the steps of reacting soluble HM1.24 antigen protein and anti-HM1.24 antibody contained in a test sample, and then detecting or determining the anti-HM1.24 antibody bound to the soluble HM1.24 antigen protein; and, an immunochemical assay for soluble HM1.24 antigen protein, said method comprising the steps of reacting anti-HM1.24 antibody and soluble HM1.24 antigen protein contained in a test sample, and then detecting or determining the soluble HM1.24 antigen protein bound to the anti-HM1.24 antibody.

## Description

### Technical Field

The present invention relates to an immunochemical assay for anti-HM1.24 antibody. The present invention also relates to an immunochemical assay for soluble HM1.24 antigen protein. Furthermore, the present invention relates to soluble HM1.24 antigen protein and DNA encoding the same.

### Background Art

Goto, T. et al. have reported mouse monoclonal antibody, anti-1.24 antibody that recognizes an antigen of molecular weight 22 to 39 kDa specifically expressed in a B cell line obtained by immunizing human plasma cells (Blood (1994) 84, 1922-1930). This mouse anti-HM1.24 antibody exhibits an in vivo anti-cancer effect in human plasma cell-implanted mice as well as an in vitro anti-cancer effect due to its ADCC (antibody-dependent cellular cytotoxicity) activity on human plasma cells (Ozaki, S. et al., Blood (1997) 90, 3179-3189).

Chimeric antibody and reshaped human antibody of this anti-HM1.24 antibody have also been created (Koichiro Ozawa et a)., The 20th Annual Meeting for The Molecular Biology Society of Japan (1997), Abstracts, general subject 3-501-P-478).

On the other hand, the determination of activity of these mouse HM1.24 antibody, chimeric antibody, and reshaped human antibody has been conducted in a cell-ELISA (Goto, T. et al. Blood (1994) 84, 1922-1930), and there is a need for more precise assay methods.

### Disclosure of the Invention

Anti-HM1.24 antibody and its antigen, i.e., HM1.24 antigen, protein expressed on the cell membrane have already been reported as described above. However, methods have not been known that detect or determine soluble HM1.24 antigen protein, low levels of soluble HM1.24 antigen protein or anti-HM1.24 antibody.

Thus, the present invention provides a simple method that detects or determines low levels of soluble HM1.24 antigen protein or anti-HM1.24 antibody.

Thus, the present invention provides (1) an immunochemical assay for anti-HM1.24 antibody, said method comprising the steps of reacting soluble HM1.24 antigen protein and anti-HM1.24 antibody contained in a test sample, and then detecting or determining the anti-HM1.24 antibody bound to the soluble HM1.24 antigen protein. The soluble HM1.24 antigen protein is preferably fused to another peptide or polypeptide. The soluble HM1.24 antigen protein is preferably bound to a support.

The support is preferably beads or a plate. The soluble HM1.24 antigen protein is bound to the support by means of antibody against soluble HM1.24 antigen protein or another peptide or polypeptide fused to soluble HM1.24 antigen protein.

The present invention also provides (2) an immunochemical assay described in the above (1), said method comprising detecting or determining the anti-HM1.24 antibody bound to the soluble HM1.24 antigen protein using a primary antibody against anti-HM1.24 antibody.

The present invention also provides (3) an immunochemical assay described in the above (1) and (2), said method comprising detecting or determining the anti-HM1.24 antibody bound to the soluble HM1.24 antigen protein using a primary antibody against the anti-HM1.24 antibody and a second antibody against the primary antibody. In the above (1) and (2), the primary antibody or the second antibody is preferably labeled with a radioisotope, an enzyme, biotin/avidin or a fluorogenic substance.

The present invention also provides (4) an immunochemical assay for soluble HM1.24 antigen protein, said method comprising the steps of reacting anti-HM1.24 antibody and soluble HM1.24 antigen protein contained in a test sample, and then detecting or determining the soluble HM1.24 antigen protein bound to the anti-HM1.24 antibody. The soluble HM1.24 antigen protein is preferably fused to another peptide or polypeptide. The anti-HM1.24 antibody is preferably bound to a support.

The support is preferably beads or a plate. The anti-HM1.24 antibody is preferably bound to the support by means of antibody against anti-HM1.24 antibody.

The present invention also provides (5) an immunochemical assay described in the above (4), said method comprising detecting or determining the soluble HM1.24 antigen protein bound to the anti-HM1.24 antibody using a primary antibody against the soluble HM1.24 antigen protein or a primary antibody against another peptide or polypeptide bout to soluble HM1.24 antigen protein.

The present invention also provides (6) an immunochemical assay described in the above (4) and (5), said method comprising detecting or determining the soluble HM1.24 antigen protein bound to the anti-HM1.24 antibody using a primary antibody against the soluble HM1.24 antigen protein or a primary antibody against another peptide or polypeptide fused to the soluble HM1.24 antigen protein, and a second antibody against the primary antibody. In the above (4) and (5), the primary antibody or the second antibody is preferably labeled with a radioisotope, an enzyme, biotin/avidin or a fluorogenic substance.

The present invention also provides (7) soluble HM1.24 antigen protein having the amino acid sequence as set forth in SEQ ID NO: 1.

The present invention also provides (8) a fusion protein of the soluble HM1.24 antigen protein described in the above (7) and another peptide or polypeptide. Specific examples of fusion proteins of soluble HM1.24 antigen protein and another peptide or polypeptide are described in SEQ ID NO: 3 and 4. The present invention also provides (9) DNA encoding the soluble HM1.24 antigen protein or a fusion protein of the soluble HM1.24 antigen protein and another peptide or polypeptide described in the above (7) and (8). DNA encoding soluble HM1.24 antigen protein or a fusion protein of soluble HM1.24 antigen protein and another peptide or polypeptide has the sequence as set forth in SEQ ID NO: 1. Other specific examples include the nucleotide sequences as set forth in SEQ ID NO: 3 and 4.

### Brief Explanation of Drawings

Fig. 1 is a schematic diagram showing the decoded range of the nucleotide sequence of a vector CGM/HA into which a gene expressing the HA tag has been inserted.
Fig. 2 is a schematic diagram showing a sandwich ELISA system that employs an HA tagged soluble antigen.
Fig. 3 is a graph showing a standard curve of humanized anti-HM1.24 antibody in a sandwich ELISA system that employs the culture supernatant of COS-7 cells in which an HA tagged soluble antigen has been transiently expressed.
Fig. 4 is a graph showing a standard curve of humanized anti-HM1.24 antibody in a sandwich ELISA system that employs the culture supernatant of COS-7 cells in which an HA tagged soluble antigen has been stably expressed.
Fig. 5 is a graph showing a time course of blood levels of antibody in Rhesus monkey that received chimeric anti-HM1.24 antibody, said level being determined by a sandwich ELISA system that employs HA tagged soluble antigen.
Fig. 6 is a graph showing that humanized anti-HM1.24 antibody inhibits the binding of biotin-labeled mouse anti-HM1.24 antibody to HA tagged soluble antigen in a dose dependent manner in a sandwich ELISA system that employs a HA tagged soluble antigen, similarly to chimeric-HM1.24 antibody.
Fig. 7 is a graph showing that the fluorescence intensity of mouse anti-HM1.24 antibody (panel to the left and anti-HA antibody (panel to the right) is shifted as compared to the control antibody (indicated by a broken line) in a FCM analysis that employs CHO cells in which HA tagged soluble antigen has been stably produced. In the figure, #1 refers to a CHO cell line selected with G418, and #A refers to a CHO cell line selected with 5 nM MTX using a #1 cell as a parent strain.
Fig. 8 is a photograph showing HM1.24 antigen detected after the culture supernatant and the cell lysate from CHO cells in which HA tagged soluble antigen has been stably produced were subjected to SDS-polyacrylamide gel electrophoresis under a reduced condition and then to Western blot using mouse anti-HM1.24 antibody. In the figure, #1 refers to a CHO cell line selected with G418; #A, #B, and #C each refer to a CHO cell line selected with 5 nM MTX using a #1 cell as a parent strain; and KPMM2 cell lysate is a positive control of HM1.24 antigen.
Fig. 9 is a photograph showing HM1.24 antigen detected after the culture supernatant and the cell lysate from CHO cell #C strain in which HA tagged soluble antigen has been stably produced were subjected to SDS-polyacrylamide gel electrophoresis under a reduced and non-reduced condition and then to Western blot using mouse anti-HM1.24 antibody. In the figure, #C refers to a CHO cell line selected with 5 nM MTX, and KPMM2 cell lysate is a positive control of HM1.24 antigen.
Fig. 10 is a schematic diagram showing the decoded range of the nucleotide sequence of a vector CGM/HA-HM164 for HA tagged C-terminal deleted soluble HM1.24 antigen.
Fig. 11 is a graph showing a standard curve of humanized anti-HM1.24 antibody in a sandwich ELISA system that employs the culture supernatant of COS-7 cells in which HA tagged C-terminal deleted soluble HM1.24 antigen has been expressed.
Fig. 12 is a graph showing a standard curve of humanized anti-HM1.24 antibody in a sandwich ELISA system that employs the culture supernatant of CHO cells in which HA tagged C-terminal deleted soluble HM1.24 antigen has been expressed.
Fig. 13 is a photograph showing HM1.24 antigen detected after the culture supernatant and the cell lysate from CHO cells (#2, #21, #28) in which HA tagged C-terminal deleted soluble antigen has been produced were subjected to SDS-polyacrylamide gel electrophoresis under a reduced condition and then to western blot using mouse anti-HM1.24 antibody. In the figure, CHO/sHM refers to a CHO cell line in which HA tagged C-terminal deleted soluble antigen, and the culture supernatant thereof has been used as a positive control of HM1.24 antigen.
Fig. 14 is a drawing that shows the amino acid sequence corresponding to the nucleotide sequence of cDNA encoding HM1.24 antigen protein.
Fig. 15 is a drawing that shows the amino acid sequence corresponding to the nucleotide sequence of cDNA encoding HM1.24 antigen protein.
Fig. 16 is a schematic diagram showing clone P3.19 isolated by the Panning method and 5 clones (IS1 to IS5) isolated by the immunoscreening method.
Fig. 17 is a drawing that shows the result of flow cytometry analysis using anti-HM1.24 antibody (A: CHO/NEO, B: CHO/HM). The histogram of anti-HM1.24 antibody is shown by a solid line, and that of the control antibody (UPC10) that showed the same isotype is shown by a broken line. In the figure, the abscissa refers to fluorescence intensity and the ordinate to cell count.
Fig. 18 a photograph in which the expression of HM1.24 antigen in each cell line and CHO cells expressing HM1.24 was detected by the immunoprecipitation/Western blotting method using anti-HM1.24 antibody. After immunoprecipitation using the anti-HM1.24 antibody-bound Sepharose 4B (lanes 1 to 6) or unbound Sepharose 4B (lanes 7 and 8), Western blotting was carried out using anti-HM1.24 antibody to detect HM1.24 antigen (shown on the right). (*: anti-HM1.24 antibody H chain).
Fig. 19 is a graph showing a standard curve of humanized anti-HM1.24 antibody in the ELISA system that employs GST-tagged soluble HM1.24 antigen expressed by Escherichia coli.

### Embodiment for Carrying Out the Invention

The soluble HM1.24 antigen protein of the present invention may be any protein as long as it has the amino acid sequence comprising Asn at amino acid position 1 to Gln at amino acid position 132 of the amino acid sequence as set forth in SEQ ID NO: 1 and it has the biological activity of soluble HM1.24 antigen protein. The biological activity of soluble HM1.24 antigen protein, as used herein, means that it can be specifically bound to anti-HM1.24 antibody, it is not bound to the cell membrane but is free from it and soluble, and it is a dimer.

The soluble HM1.24 antigen protein of the present invention may also be any soluble HM1.24 antigen protein that has the biological activity of soluble HM1.24 antigen protein, and has the amino acid sequence which has been modified by substitution, deletion and/or addition of one or a plurality of amino acids in the amino acid sequence as set forth in SEQ ID NO: 1. More specifically, the soluble HM1.24 antigen protein of the present invention may have amino acids in which one or more than one, preferably one or not greater than 24, and more preferably one or not greater than 12 amino acid residues have been substituted in the amino acid sequence as set forth in SEQ ID NO: 1.

Alternatively, the amino acid sequence as set forth in SEQ ID NO: 1 may be modified by deletion of one or more than one, preferably one or not greater than 42, and more preferably one or not greater than 17 amino acid residues. Alternatively, the amino acid sequence as set forth in SEQ ID NO: 1 may be modified by the addition of one or more than one, preferably one or not greater than 50, and more preferably one or not greater than 14, amino acid residues. The soluble HM1.24 antigen protein for use in the present invention may have undergone modification by the above substitution, deletion, and/or addition at the same time.

Soluble HM1.24 antigen protein has been demonstrated to exhibit its biological activity as long as it has the amino acid sequence comprising amino acid Asn at position 1 to amino acid Arg at position 90 in SEQ ID NO: 1. The soluble HM1.24 antigen protein of the present invention may be a soluble HM1.24 antigen protein that has the amino acid sequence from amino acid Asn at position 1 to amino acid Arg at position 90 in SEQ ID NO: 1, or the amino acid sequence modified by the substitution, deletion, and/or addition of one or a plurality of amino acid residues in the amino acid sequence from amino acid Asn at position 1 to amino acid Arg at position 90.

Soluble HM1.24 antigen protein may be a soluble HM1.24 antigen protein that has the amino acid sequence from amino acid Arg at position 90 to amino acid Gln at position 132 in SEQ ID NO: 1, or the amino acid sequence modified by the substitution, deletion, and/or addition of one or a plurality of amino acid residues to this amino acid sequence, as long as it has the biological activity.

As a soluble HM1.24 antigen protein having the amino acid sequence modified by the substitution, deletion, and/or addition of one or a plurality of amino acid residues in the amino acid sequence of SEQ ID NO: 1, there may be mentioned a soluble HM1.24 antigen protein having the amino acid sequence as set forth in SEQ ID NOs: 3 and 4.

It has already been known that a protein having an amino acid sequence modified by the substitution, deletion, and/or addition of one or a plurality of amino acid residues to a given amino acid sequence maintains its biological properties (Mark, D.F. et al., Proc. Natl. Acad. Sci. U.S.A. (1984) 81, 5662-5666, Zoller, M.J. & Smith, M. Nucleic Acids Research (1982) 10, 6487-6500, Wang et al., Science 224, 1431-1433, Dalbadie-McFarland, G. et al., Proc. Natl. Acad. Sci. U.S.A. (1982) 79, 6409-6413).

For soluble HM1.24 antigen proteins of the present invention, the amino acid sequences, molecular weight, isoelectric point, the presence and position of sugar chain addition, the structure of a sugar chain, the state of phosphorylation and/or the presence of a disulfide bond may vary depending on the species from which they are obtained, the host that produces them, and/or the method of purification. However, proteins of any structure may be used as long as it can be preferably used in the present invention. The species from which the proteins are obtained is preferably human.

As DNA encoding the soluble HM1.24 antigen proteins of the present invention, there can be mentioned a nucleotide sequence from the necleotide adenine at the nucleotide position 1 to the guanine at the position 396 as set forth in SEQ ID NO: 1. As DNA encoding the soluble HM1.24 antigen proteins of the present invention, DNA of any origin may be used as long as it has the nucleotide sequence as set forth in SEQ ID NO: 1. One example of such DNA includes, for example, genomic DNA, cDNA, and synthetic DNA. The DNA may be DNA obtained from a cDNA library or a genomic library obtained from various cells, tissues, or organs, or from species other than humans, and they may be from a commercially available DNA library. Vectors for use in such libraries may be plasmids, bacteriophages, YAC vectors, and the like.

DNA encoding soluble HM1.24 antigen protein for use in the present invention may be DNA that hybridizes to the nucleotide sequence as set forth in SEQ ID NO: 1 and encodes a protein having the biological activity of soluble HM1.24 antigen protein. As a condition under which DNA encoding soluble HM1.24 antigen protein hybridizes, there may be mentioned a suitable stringent condition.

Such conditions include, for example, a low stringent condition. By way of example, a low stringent condition is a washing condition provided by room temperature in 2 x SSC and 0.1% sodium dodecyl sulfate. More preferably, there may be mentioned a high stringent condition. By way of example, a high stringent condition is a washing condition provided by 60°C, in 0.1 x SSC and 0.1% sodium dodecyl sulfate. It is already known that a protein encoded by a DNA that hybridizes under a suitable stringent condition to a nucleotide sequence encoding protein has the same biological activity as the protein.

Thus, the soluble HM1.24 antigen protein of the present invention is encoded by the above "DNA that hybridizes" and includes proteins having the biological activity of soluble HM1.24 antigen protein.

The amino acid sequence of human HM1.24 antigen protein expressed on the cell membrane is shown in SEQ ID NO: 16. E. coli that has the plasmid pRS38-pUC19 having DNA encoding human protein having an amino acid sequence as set forth in SEQ ID NO: 16 at the XbaI cleavage site was designated as Escherichia coli DH5α (pRS38-pUC19) and has been internationally deposited under the provisions of the Budapest Treaty on October 5, 1993, with the National Institute of Bioscience and Human Technology, Agency of Industrial Science and Technology, of 1-3, Higashi 1-chome, Tsukuba-shi, Ibaraki pref., Japan, under accession number FERM BP-4434.

The soluble HM1.24 antigen proteins of the present invention may be the above proteins that are fused to another peptide or polypeptide, as long as they have the biological activity of soluble HM1.24 antigen protein. Such fusion proteins may be produced by a known method. Another peptide or polypeptide subjected to fusion with the protein may be any peptide or polypeptide as long as it can be advantageously used in the present invention. As such peptides, for example, known peptides may be used including FLAG (Hopp, T. P. et al., BioTechnology (1988) 6, 1204-1210), 6 x His comprising 6 His (histidine) residues, 10 x His, influenza hemagglutinin (HA), fragments of human c-myc, fragments of VSV-GP, fragments of p18HIV, T7-tag, HSV-tag, E-tag, fragments of SV40T antigen, lck tag, fragments of a-tubulin, B-tag, fragments of Protein C, and the like.

As polypeptides, there may be mentioned, for example, GST (glutathione S-transferase), HA, the constant regions of immunoglobulin, β-galactosidase, MBP (maltose-binding protein), and the like. They may be commercially available polypeptides.

DNA encoding the protein for use in the present invention may be generated by constructing the above-mentioned DNA using commercially available kits or by known methods. Construction may be effected, for example, by digestion with a restriction enzyme, addition of a linker, insertion of an initiation codon (ATG) and/or a stop codon (ATT, TGA or TAG), and the like.

Expression vectors for use in the present invention may be any expression vectors as long as they can be suitably used in the present invention. As expression vectors, there may be mentioned expression vectors derived from a mammal such as pEF and pCDM8, expression vectors derived from an insect such as pBacPAK8, expression vectors derived from a plant such as pMH1 and pMH2, expression vectors derived from an animal virus such as pHSV and pMV, expression vectors derived from a yeast such as pNV11, expression vectors derived from Bacillus subtilis such as pPL608 and pKTH50, and expression vectors derived from Escherichia coli such as pGEX, pGEMEX and pMALp2.

Expression vectors of proteins for use in the present invention may be produced by linking DNA encoding soluble HM1.24 antigen protein downstream to the promoter. As promoters/enhancers, promoters/enhancers derived from a mammal such as the EF1-α promoter/enhancer and γ-actin promoter/enhancer, promoters/enhancers derived from an insect such as polyhedrin virus promoter/enhancer, promoters/enhancers derived from a plant such as tabacco mosaic virus promoter/enhancer, promoters/enhancers derived from a plant such as SV40 promoter/enhancer and human CMV promoter/enhancer, promoters/enhancers derived from yeast such as alcohol dehydrogenase promoter/enhancer, promoters/enhancers derived from Escherichia coli such as Lac promoter/enhancer, Trp promoter/enhancer and Tac Promoter/enhancer may be used.

For the expression of protein for use in the present invention, a signal sequence suitable for the host to be used in the expression may be added. As the signal sequence, there may be mentioned a signal sequence for secretary proteins. As a signal sequence for secretary proteins, there may be mentioned a signal sequence for secretary proteins derived from a mammal such as a signal sequence for immunoglobulins. As a signal sequence for secretary proteins, there may be mentioned a signal sequence for secretary proteins derived from E. coli such as periplasm secretary signal sequence such as OmpA.

An expression vector produced as mentioned above can be introduced into a host by a known method. Methods for introduction into the host includes, for example, electroporation, the calcium phosphate method, and the liposome method.

Proteins for use in the present invention can be obtained as recombinant proteins produced using gene recombinant technology as described above. For example, recombinant proteins may be produced by cloning the nucleotide sequence of a gene described herein from a cell, tissue, or an organ that expresses them and integrating the gene into a suitable vector, which is introduced into a host to allow the host to produce said proteins. The recombinant proteins can be used in the present invention.

Specifically, mRNA encoding a gene can be isolated from the cell, tissue or organ that expresses proteins to be used in the present invention. The isolation of mRNA is conducted by preparing total RNA using a known method such as the guanidine ultracentrifugation method (Chirgwin, J. M. et al., Biochemistry (1979) 18, 5294-5299), the AGPC method (Chomzynski, P. and Sacci, N., Anal. Biochem. (1987) 162, 156-159), and then purifying mRNA from the total RNA using the mRNA Purification Kit (Pharmacia) and the like. Alternatively, mRNA can be prepared directly using the QuickPrep mRNA Purification kit (Pharmacia).

An mRNA obtained is used to synthesize cDNA of the gene using a reverse transcriptase. The synthesis of cDNA can be effected using an AMV Reverse Transcriptase First-strand cDNA Synthesis Kit (Seikagaku Kogyo), and the like. Alternatively, for the synthesis and amplification of cDNA, a Marathon cDNA Amplification kit (manufactured by CLONTECH) and 5'-RACE method (Frohman, M.A. et al., Proc. Natl. Acad. Sci. U.S.A. (1988) 85, 8998-9002; Belyavsky, A. et al., Nucleic Acids Res. (1989) 17, 2919-2932) that employs the polymerase chain reaction (PCR) may be used.

A DNA fragment of interest may be prepared from the PCR product thus obtained and ligated to a vector DNA. Furthermore, a recombinant vector is constructed, from this, and is then introduced into E. coli for selection of colonies to prepare the desired recombinant vector. The nucleotide sequence of the desired DNA may be confirmed by a known method such as the dideoxy nucleotide chain termination method. Once the desired DNA has been obtained, it may be integrated into an expression vector.

More specifically, the DNA constructed as described above may be expressed to obtain protein. When mammalian cells are used, expression may be accomplished using a conventionally used useful promoter/enhancer, the gene to be expressed, and DNA in which the poly A signal has been operably linked at 3' downstream thereof or a vector containing them. Examples of the promoter/enhancer include a human cytomegalovirus immediate early promoter/enhancer.

Additionally, as the promoter/enhancer which can be used for expression thereof, there are viral promoters/enhancers such as retrovirus, polyoma virus, adenovirus, and simian virus 40 (SV40), and promoters/enhancers derived from mammalian cells such as human elongation factor 1α (HEF1α).

For example, expression may be readily accomplished by the method of Mulligan et al. (Nature (1979) 277, 108) when the SV40 promoter/enhancer is used, or by the method of Mizushima et al. (Nucleic Acids Res. (1990) 18, 5322) when the HEF1α promoter/enhancer is used.

In the case of E. coli, expression may be conducted by operably linking a conventionally used useful promoter, a signal sequence for protein secretion, and the gene to be expressed, followed by expression thereof. As the promoter, for example, there can be mentioned the lacz promoter and the araB promoter. The method of Ward et al. (Nature (1098) 341, 544-546; FASEB J. (1992) 6, 2422-2427) may be used when the lacz promoter is used, and the method of Better et al. (Science (1988) 240, 1041-1043) may be used when the araB promoter is used.

As the signal sequence for protein secretion into the periplasm of E. coli, the pelB signal sequence (Lei, S. P. et al., J. Bacteriol. (1987) 169, 4379) can be used.

As the origin of replication, there can be used those derived from SV40, polyoma virus, adenovirus, bovine papilloma virus (BPV) and the like. Furthermore, for the amplification of gene copy number in the host cell system, expression vectors can include, as selectable markers, the aminoglycoside transferase (APH) gene, the thymidine kinase (TK) gene, E. coli xanthine guaninephosphoribosyl transferase (Ecogpt) gene, the dihydrofolate reductase (dhfr) gene and the like.

For the production of proteins for use in the present invention, any production system can be used. The production system of protein preparation comprises the in vitro or the in vivo production system. As the in vitro production system, there can be mentioned a production system which employs eukaryotic cells and the production system which employs prokaryotic cells.

When the eukaryotic cells are used, there are the production systems which employ animal cells, plant cells, and fungal cells. Known animal cells include (1) mammalian cells such as CHO cells (J. Exp. Med. (1995) 108, 945), COS cells, myeloma cells, baby hamster kidney (BHK) cells, HeLa cells, and Vero cells, (2) amphibian cells such as Xenopus oocytes (Valle, et al., Nature (1981) 291, 358-340), or (3) insect cells such as sf9, sf21, and Tn5. As the CHO cells, dhfr-CHO (Proc. Natl. Acad. Sci. U.S.A. (1968) 77, 4216-4220), a CHO cell that is deficient in the DHFR gene, and CHO K-1 (Proc. Natl. Acad. Sci. U.S.A. (1968) 60, 1275) can be preferably used.

Known plant cells include, for example, those derived from Nicotiana tabacum, which is subjected to callus culture. Known fungal cells include yeasts such as the genus Saccharomyces, more specifically Saccharomyces cereviceae, or filamentous fungi such as the genus Aspergillus, more specifically Aspergillus niger.

When the prokaryotic cells are used, there are the production systems which employ bacterial cells. Known bacterial cells include Escherichia coli (E. coli), and Bacillus subtilis.

By transforming these cells with a desired DNA and culturing the transformed cells in vitro, proteins can be obtained. Culturing may be conducted in the known methods. For example, as the culture liquid, DMEM, MEM, RPMI1640, and IMDM can be used, and serum supplements such as fetal calf serum (FCS) may be used in combination, or serum-free medium can be used. pH during the culture is preferably about 6 to 8. Culture is usually carried out at 30 to 40°C for about 15 to 200 hours with, as desired, medium change, aeration, and agitation.

On the other hand, as in vivo production systems, there can be mentioned those which employ animals and those which employ plants. DNA of interest is introduced into these animals or plants, and the proteins are produced in such animals or plants, and recovered.

When animals are used, there are the production systems which employ mammals and insects.

As mammals, goat, pigs, sheep, mice, and cattle can be used (Vicki Glaser, SPECTRUM Biotechnology Applications, 1993). When mammals are used, transgenic animals can be used.

For example, the DNA of interest is inserted into the middle of the gene encoding a protein which is inherently produced in the milk such as goat β casein to prepare fusion genes. A DNA fragment containing a fusion gene into which the DNA of interest has been inserted is injected into a goat embryo, and the embryo is introduced into a female goat. The protein is obtained from the milk produced by the transgenic goat borne to the goat who received the embryo or offspring thereof. In order to increase the amount of milk containing the protein produced by the transgenic goat, hormones may be given to the transgenic goat as appropriate. (Ebert, K.M. et al., Bio/Technology (1994) 12, 699-702).

As an insect, silkworms may be used. When silkworms are used, baculovirus into which the DNA of interest has been inserted is infected to the silkworm, and the desired protein can be obtained from the body fluid of the silkworm (Susumu, M. et al., Nature (1985) 315, 592-594).

Moreover, when plants are used, tabacco, for example, can be used. When tabacco is used, the DNA of interest is inserted into an expression vector for plant, for example pMON 530, and then the vector is introduced into a bacterium such as Aprobacterium tumefaciens. The bacterium is then infected to tobacco such as Nicotiana tabacum to obtain the desired protein from the leaves of the tabacco (Julian, K.-C. Ma et al., Eur. J. Immunol. (1994) 24, 131-138).

As methods of introducing an expression vector into a host, there can be used a known method such as the calcium phosphate method (Virolgoy (1973) 52, 456-467), the electroporation method (EMBO J. (1982) 1, 841-845), and the like. Considering the frequency of use of the host's codon for use in the present invention, a sequence having a better efficiency of expression can be designed (Grantham, R. et al., Nucleic Acids Research (1981) 9, r43-r74).

The gene is introduced as described above into these animals or plants, and protein is produced in the body of the animals or the plants and recovered. Protein expressed and produced as described above can be separated from the inside or outside of the host cell and then may be purified to homogeneity. Separation and purification of the antibody for use in the present invention may be accomplished by, but this is not limited to, the separation and the purification methods conventionally used for protein purification.

Proteins can be separated and purified by selecting and combining, as appropriate, methods including, but not limited to, chromatography columns such as affinity chromatography, filtration, ultrafiltration, salting-out, dialysis, SDS-polyacrylamide gel electrophoresis, isoelectric focusing, and the like (Shinseikagaku Jikken Koza I [A New Course in Experimental Biochemistry I], 1990, Tokyo Kagaku Dojin).

As chromatography, there may be mentioned, for example, affinity chromatography, ion exchange chromatography, hydrophobic chromatography, gel-filtration, reverse phase chromatography, adsorption chromatography, and the like (Strategies for Protein Purification and Characterization: A Laboratory Course Manual. Ed Daniel R. Marshak et al., Cold Spring Harbor Laboratory Press, 1986). These chromatographies can be carried out using a liquid chromatography such as HPLC and FPLC.

Proteins can be determined using a known method. For example, measurement of absorbance or the Bradford method can be used.

The present invention relates to an immunochemical assay for anti-HM1.24 antibody, said method comprising reacting soluble HM1.24 antigen protein and anti-HM1.24 antibody contained in a test sample, and then detecting or measuring the anti-HM1.24 antibody bound to the soluble HM1.24 antigen protein; and
an immunochemical assay for soluble HM1.24 antigen protein, said method comprising reacting anti-HM1.24 antibody and soluble HM1.24 antigen protein contained in a test sample, and then detecting or determining the soluble HM1.24 antigen protein bound to the anti-HM1.24 antibody.

The immunochemical assay for use in the present invention may be conducted as an in vitro assay system.

The in vitro assay system may be conducted in a non-cellular system. Specifically, soluble HM1.24 antigen protein is immobilized to a support, to which protein is added a test sample containing anti-HM1.24 antibody, and the support is incubated and then washed followed by detection or measurement of binding of the anti-HM1.24 antibody to the soluble HM1.24 antigen protein immobilized to the support. Alternatively, anti-HM1.24 antibody is immobilized to a support, to which a test sample containing soluble HM1.24 antigen protein is added. After incubation and washing, the binding of the soluble HM1.24 antigen protein to the anti-HM1.24 antibody immobilized to the support may be detected or measured.

Soluble HM1.24 antigen protein or anti-HM1.24 antibody may be a protein produced by cells that inherently express them, cells into which DNA encoding it has been introduced, or animals or plants into which DNA encoding it has been introduced, which protein may be used in a purified or crude-purified form.

One of the purified or crude-purified soluble HM1.24 antigen protein or anti-HM1.24 antibody is allowed to be immobilized to the support. The protein may be immobilized onto the support by a standard method in biding proteins to supports. As supports to which proteins are bound, there may be mentioned, for example, insoluble polysaccharides such as agarose, dextran, cellulose, synthetic resin such as polystyrene, polyacrylamide and silicone.

More specifically, commercially available beads, or a plate, that are produced using the above as a raw material are used. In the case of beads, there may be used columns that are packed with them. In the case of plates, there may be mentioned multi-well plates (96-well multi-well plates, etc.) or biosensor chips.

Immobilization between proteins and supports may be effected using conventionally known methods such as chemical bonding and physical adsorption. Alternatively, it is be possible to bind an antibody that specifically recognizes the protein to a support in advance so that the antibody and the protein become joined. Furthermore, avidin/biotin can also be bound.

The binding between soluble HM1.24 antigen protein and anti-HM1.24 antibody may usually be effected in a buffer solution. As buffer solutions, for example, phosphate buffers, Tris buffers and the like may be used. Incubation conditions may be any conditions that are commonly used, including the incubation at 4°C to room temperature for 1 hour to 24 hours. Washing after the incubation may be effected in any solution that does not prevent binding between soluble HM1.24 antigen protein and anti-HM1.24 antibody including, for example, a buffer solution containing a surfactant. As surfactants, 0.05% Tween 20 may be used.

Test samples that contain soluble HM1.24 antigen protein or anti-HM1.24 antibody include, for example, human fluids (blood, serum, urine, synovial fluids, etc.), culture supernatants of cells, animal secretions (milk etc.), and pharmaceutical formulations.

When the binding of anti-HM1.24 antibody or soluble HM1.24 antigen protein to soluble HM1.24 antigen protein or anti-HM1.24 antibody is detected or measured, they are incubated and washed under an appropriate condition to separate the specific binding from the non-specific binding. Then the status of binding of soluble HM1.24 antigen protein and anti-HM1.24 antibody can be evaluated.

In the immunochemical assay of the present invention, control groups can be set up together with the group in which the test samples are contacted to the proteins. As a control group, the negative control group having no test samples, the positive control group having a standard of soluble HM1.24 antigen protein or anti-HM1.24 antibody, or both of the groups can be set up.

In accordance with the immunochemical assay of the present invention, the bound protein can be detected, or the bound protein may be measured in a quantitative manner. In these cases, the result obtained for the negative control group having no test samples, the result obtained for the group having a test sample, and/or the result obtained for the positive control group having a standard of soluble HM1.24 antigen protein or anti-HM1.24 antibody can be compared to detect binding between soluble HM1.24 antigen protein and anti-HM1.24 antibody.

Alternatively, these results may be obtained in numerical values, which values may be compared to quantify the soluble HM1.24 antigen protein or anti-HM1.24 antibody contained in test samples. When quantitative determination is made, the numerical value obtained with the negative control group having no test sample and that obtained with the group in which a test sample that contains soluble HM1.24 antigen protein or anti-HM1.24 antibody was applied may be compared to quantify the amount of binding between soluble HM1.24 antigen protein and anti-HM1.24 antibody. If soluble HM1.24 antigen protein or anti-HM1.24 antibody is contained in a test sample, soluble HM1.24 antigen protein or anti-HM1.24 antibody would be detected or measured by the presence of the bound protein.

When quantitative determinations are also made, quantitation may be made based on a standard curve generated from the numerical values obtained with the positive control group containing known amounts of soluble HM1.24 antigen protein or anti-HM1.24 antibody.

In a immunochemical assay of the present invention, biosensors may be used that utilize a surface plasmon resonance phenomenon as a means to detect or measure soluble HM1.24 antigen protein or anti-HM1.24 antibody in test samples. Biosensors that utilize a surface plasmon resonance phenomenon permit a real time observation of protein-protein interaction as a surface plasmon resonance signal using and without labeling a trace amount of protein (for example BIAcore; manufactured by Pharmacia). Thus, by using biosensors such as BIAcore, binding between soluble HM1.24 antigen protein and anti-HM1.24 antibody can be evaluated.

Specifically, by contacting a test sample containing anti-HM1.24 antibody or soluble HM1.24 antigen protein to a sensor chip on which is immobilized soluble HM1.24 antigen protein or anti-HM1.24 antibody, anti-HM1.24 antibody or soluble HM1.24 antigen protein bound to soluble HM1.24 antigen protein or anti-HM1.24 antibody can be detected or determined as changes in resonance signals.

More specifically it may be carried out as follows. First a sensor chip CM5 (Biosensor) is activated and then soluble HM1.24 antigen protein or anti-HM1.24 antibody is immobilized thereon. Thus, after the sensor chip is activated with an aqueous solution of EDC/NHS (200 mM EDC (N-ethyl-N'-(3-dimethylaminopropyl)carbonate, hydrochloride) and 50 mM NHS (N-hydroxysuccinimide)), it is washed with an HBS buffer (10 mM HEPES, pH 7.4, 150 mM NaCl, 3.4 mM EDTA, 0.05% Tween 20).

Then a test sample containing an appropriate amount of anti-HM1.24 antibody or soluble HM1.24 antigen protein dissolved in the HBS buffer is contacted to the sensor chip and immobilized thereon. After washing the sensor chip with the HBS buffer, active groups remaining on the sensor chip are blocked with an ethanolamine solution (1M ethanolamine hydrochloride, pH 8.5). The sensor chip is washed again with the HBS buffer for use in the evaluation of binding.

Then an appropriate amount of test sample containing anti-HM1.24 antibody or soluble HM1.24 antigen protein dissolved in the HBS buffer is injected, whereupon the amount of anti-HM1.24 antibody or the soluble HM1.24 antigen protein that is bound to the soluble HM1.24 antigen protein or the anti-HM1.24 antibody immobilized on the sensor chip is observed as an increment in the value of resonance signal.

Furthermore, control groups may be set up together with test samples. AS control groups, the negative control group having no test samples, the positive control group having a known amount of soluble HM1.24 antigen protein or anti-HM1.24 antibody, or both of the groups can be set up. The bound protein can be quantitatively determined as a change in the value of resonance signal. In these cases, the result obtained for the negative control group having no test samples, the result obtained for the group having a test sample, and/or the result obtained for the positive control group having a known amount of soluble HM1.24 antigen protein or anti-HM1.24 antibody can be compared to detect or determine the protein of interest.

As a means of detecting or determining the bound protein in the test sample in the immunochemical assay of the present invention, a primary antibody that specifically recognizes soluble HM1-24 antigen protein or anti-HM1.24 antibody can be used.

For example, a test sample is contacted to soluble HM1.24 antigen protein or anti-HM1.24 antibody, and after washing, the bound protein is detected or determined by means of a primary antibody that specifically recognizes the protein. Thus, preferably, to one protein that has been bound to a support is contacted a test sample containing the other protein. After incubating and washing, the bound protein can be detected or determined by a primary antibody that specifically recognizes the protein. The primary antibody is preferably labeled with a labeled substance.

Soluble HM1.24 antigen protein may be fused to another peptide or polypeptide. Thus, in order to detect soluble HM1.24 antigen protein contained in the test sample, anti-HM1.24 antibody can be used, or antibody to another peptide or polypeptide fused to soluble HM1.24 antigen protein can be used. Alternatively, in order to detect anti-HM1.24 antibody contained in the test sample, antibody that specifically recognizes anti-HM1.24 antibody can be used. When anti-HM1.24 antibody is a mouse antibody, anti-mouse immunoglobulin antibody can be used as an antibody that specifically recognizes anti-HM1.24 antibody. Alternatively, when anti-HM1.24 antibody is a chimeric antibody or a humanized antibody, anti-human immunoglobulin antibody can be used as an antibody that specifically recognizes anti-HM1.24 antibody.

Protein can be labeled by commonly known methods. As labels, there may be used, for example, radioisotopes, enzymes, fluorescent substances, biotin/avidin, and the like. These labels may be commercially available. As radioisotopes, there may be mentioned, for example, ³²P, ³³P, ¹³¹I, ¹²⁵I, ³H, ¹⁴C, and ³⁵S. As enzymes, there may be mentioned, for example, alkaline phosphatase, horseradish peroxidase, β-galactosidase, β-glucosidase, and the like. As fluorescent substances, there may be mentioned, for example, fluorescein isocyanate (FITC) and rhodamine. These are commercially available and may be labeled by known methods.

Specifically, the following procedure may be used. Thus, a solution containing soluble HM1.24 antigen protein or anti-HM1.24 antibody is added to a plate, which is then allowed to stand overnight to effect immobilization. When soluble HM1.24 antigen protein or anti-HM1.24 antibody is immobilized, antibody against each of them may be immobilized in advance, and then soluble HM1.24 antigen protein or anti-HM1.24 antibody may be bound to the immobilized antibody. After washing the plate, it is blocked with, for example, BSA to prevent non-specific binding of proteins. The plate is washed again, and a test sample containing anti-HM1.24 antibody or soluble HM1.24 antigen protein is added to the plate. At the same time, a group (the negative control) containing no test samples and/or a group (the positive control) to which a known concentration of anti-HM1.24 antibody or soluble HM1.24 antigen protein has been added are set up and incubated.

After incubation, it is washed and antibody against the test sample is added. After appropriate incubation, the plate is washed and the protein is detected or measured by a primary antibody that specifically recognizes the protein. For the detection or measurement, in the case of a radioisotope, liquid scintillation is used. In the case of an enzyme, the substrate is added and an enzymatic change, for example color development of the substrate, is detected or measured. In the case of a fluorogenic substance, a fluorospectrophotometer is used for detection or measurement. Comparison of these results with the numerical value obtained for the control group permits the identification of the test sample containing the inhibiting substance.

As a means of detecting or measuring soluble HM1.24 antigen protein or anti-HM1.24 antibody in the test sample, a primary antibody that specifically recognizes soluble HM1.24 antigen protein or anti-HM1.24 antibody or a second antibody that specifically recognizes the primary antibody may be used.

For example, in the above immunochemical assay, a test sample is brought to contact with a soluble HM1.24 antigen protein or anti-HM1.24 antibody, incubated, washed, and then the bound protein is detected or measured by means of a primary antibody that specifically recognizes the protein and a second antibody that specifically recognizes the primary antibody. Thus, specifically, soluble HM1.24 antigen protein or anti-HM1.24 antibody is immobilized to a support, to which is contacted the test sample. After incubating and washing, the bound protein may be detected or measured by means of a primary antibody that specifically recognizes the protein or a second antibody that specifically recognizes the primary antibody. Preferably, the second antibody is labeled with a label. The antibody can be labeled by the above known methods.

Specifically, the following procedure may be used. Thus, a solution containing soluble HM1.24 antigen protein or anti-HM1.24 antibody is added to a plate, which is then allowed to stand overnight to effect immobilization. When immobilized to the plate, antibody against each of them may be immobilized in advance, and then soluble HM1.24 antigen protein or anti-HM1.24 antibody may be bound to the immobilized antibody. After washing the plate, it is blocked with, for example, BSA to prevent the non-specific binding of proteins. The plate is washed again, and a test sample is added to the plate. At the same time, a group (the negative control) containing no test samples and/or a group (the positive control) to which a known concentration of a anti-HM1.24 antibody or soluble HM1.24 antigen protein has been added are set up and incubated.

After incubation, the plate is washed and a primary antibody against anti-HM1.24 antibody or soluble HM1.24 antigen protein contained in the test sample is added. After appropriate incubation, the plate is washed and a second antibody that specifically recognizes the primary antibody is added. After appropriate incubation, the plate is washed, and the protein is detected or determined by a second antibody that specifically recognizes the primary antibody. For the detection or determination, in the case of a radioisotope, liquid scintillation is used. In the case of an enzyme, the substrate is added and an enzymatic change, for example color development of the substrate, is detected or measured. In the case of a fluorogenic substance, a fluorospectrophotometer is used for detection or measurement.

Comparison of these results with the numerical value obtained for the control group permits the identification of a test sample containing inhibiting substances. Soluble HM1.24 antigen protein may be fused to another peptide or polypeptide. Thus, as a primary antibody for detecting soluble HM1.24 antigen protein contained in the test sample, anti-HM1.24 antibody can be used as a primary antibody, or antibody against another peptide or polypeptide that is fused to soluble HM1.24 antigen protein can be used. Furthermore, antibody that specifically recognizes anti-HM1.24 antibody can be used in order to detect anti-HM1.24 antibody contained in the test sample.

When anti-HM1.24 antibody is a mouse antibody, anti-mouse immunoglobulin antibody can be used as an antibody that specifically recognizes anti-HM1.24 antibody. Alternatively, when anti-HM1.24 antibody is a chimeric antibody or a humanized antibody, anti-human immunoglobulin antibody can be used as a primary antibody that specifically recognizes anti-HM1.24 antibody. Alternatively, antibody that specifically recognizes the primary antibody can be used as a second antibody. For example, when the primary antibody is a sheep antibody, anti-sheep immunoglobulin antibody can be used. Alternatively, when the primary antibody is a rabbit antibody, anti-rabbit immunoglobulin antibody can be used.

More specifically, the present invention may be conducted by, most specifically, an ELISA (enzyme-linked immunosorbent assay). Thus, antibody against HA (influenza agglutinin) fused to soluble HM1.24 antigen protein is diluted in the immobilization buffer (0.1 M NaHCO₃, 0.02% NaN₃, pH 9.6). A suitable amount of the aqueous solution that was diluted is added to each well of a 96-well immunoplate (manufactured by Nunc), which is then incubated overnight at 4°C to effect immobilization.

After each well is washed three times with the wash buffer (prepared as 0.05% Tween 20 in PBS), 200 ml of a 5% solution of BSA (manufactured by SIGMA) dissolved in PBS is added to block at room temperature for 2 hours.

Then, each well is washed three times with the wash buffer, and the soluble HM1.24 antigen protein fused to HA diluted with the dilution buffer (1% BSA, 0.5% Tween 20, PBS) is added thereto and incubated at 4°C overnight to bind the anti-HA antibody and the soluble HM1.24 antigen protein fused to HA. After washing three times with the wash buffer, a fixed amount of a test sample containing chimeric anti-HM1.24 antibody having the constant region (C region) of human IgG antibody is added, and incubated at room temperature for 1 hour.

Each well is washed three times with the wash buffer, and 100 µl of alkaline phosphatase-labeled goat anti-human IgG antibody (manufactured by IBI) diluted 5000-fold in the dilution buffer is added to each well and incubated at room temperature for one hour. Each well is washed five times with the wash buffer, and 100 µl of the color development solution (substrate buffer; Sigma 104 dissolved to 1 mg/ml in 50 mM NaHCO₃, 10 mM MgCl₂, pH 9,8) is added to each well and incubated at room temperature. Subsequently, absorbance at 405 nm is measured using a microplate reader (Model 3550, manufactured by BIO-RAD). Comparison of these results with the numerical value obtained for the negative control group and/or the positive control group permits the detection or determination of chimeric anti-HM1.24 antibody. A similar manner can also be used to detect or determine soluble HM1.24 antigen protein.

The screening method of the present invention may also be used for High Throughput Screening (HTS). Specifically, steps up to the blocking may be conducted manually, and the subsequent reactions can be automated by robotization to realize High Throughput Screening.

Thus, antibody against HA is diluted in the immobilization buffer (0.1 M NaHCO₃, 0.02% NaN₃, pH 9.6). A suitable amount of the aqueous solution that was diluted to each well of a 96-well immunoplate (manufactured by Nunc) is added and then incubated overnight at 4°C to effect immobilization.

After each well is washed three times with the wash buffer (prepared to 0.05% Tween 20 in PBS), 200 µl of a 5% BSA solution (manufactured by SIGMA) dissolved in PBS is added to block overnight at room temperature. Subsequently, each well is washed three times with the wash buffer, to which the soluble HM1.24 antigen protein fused to HA diluted with the dilution buffer (1% BSA, 0.5% Tween 20, PBS) is added and incubated at 4°C overnight to bind anti-HA antibody and the soluble HM1.24 antigen protein fused to HA.

Subsequently, the immunoplate is mounted to, for example, the Biomek 2000 HTS system (manufactured by Beckman) and the control program of the system is executed to add a test sample containing chimeric anti-HM1.24 antibody, a primary antibody against chimeric anti-HM1.24 antibody, and a second antibody against the primary antibody.

At this time the delivery of the solution to each well of the immunoplate and the removal thereof can be carried out using the Biomek 2000 dispenser (manufactured by Beckman) or the Multipipette 96-well simultaneous dispenser (manufactured by Sagian) as a dispensing instrument. Washing of each well of the immunoplate can also be carried out using the EL404 microplate washer (Bio Tek). Measurement of absorbance can be made using the SPECTRAmax250 plate reader (manufactured by Molecular Devices).

The program is set so as to perform the following steps. Thus, each well is washed three times with the wash buffer, an fixed amount of the test sample and the test sample containing chimeric anti-HM1.24 antibody diluted in the dilution buffer (1% BSA, 0.5% Tween 20, PBS) are added. At the same time, a group (the negative control) containing no test samples and a group (the positive control) to which a known concentration of chimeric anti-HM1.24 antibody has been added are set up and incubated at room temperature for one hour. Each well is washed three times with the wash buffer, 100 µl of rabbit anti-human IgG antiserum (manufactured by New England Biolabs) diluted 5000-fold in the dilution buffer is added to each well, and incubated at room temperature for one hour. Each well is washed three times with the wash buffer, 100 µl of alkaline phosphatase-labeled goat anti-rabbit IgG antibody (manufactured by TAGO) diluted 5000-fold in the dilution buffer is added to each well, and incubated at room temperature for one hour.

Each well is then washed five times with the wash buffer, 100 µl of the color development solution (substrate buffer; p-nitrophenyl phosphate dissolved to 1 mg/ml in 50 mM NaHCO₃, 10 mM MgCl₂, pH 9,8, manufactured by Sigma) is added to each well, and incubated at room temperature. Subsequently, absorbance at 405 nm is measured using a microplate reader, the Biomek plate reader (manufactured by Beckman/Molecular Devices). Comparison of these results with the numerical value obtained for the control group permits the detection or determination of chimeric anti-HM1.24 antibody contained in the test sample. In a similar manner, soluble HM1.24 antigen protein can also be detected or determined.

The immunochemical assay of the present invention can measure soluble HM1.24 antigen protein or anti-HM1.24 antibody up to a concentration of 500 pg/ml.

Antibodies for use in the present invention may be those that are commercially available or that are contained in commercially available kits, or they can be obtained as monoclonal or polyclonal antibodies using known methods.

Monoclonal antibodies can be obtained by using the desired sensitizing antigen, which is used in a conventional method for immunization, by fusing the immune cells thus obtained with known parent cells, and screening monoclonal antibody-producing cells using a known screening method.

Specifically, monoclonal or polyclonal antibodies may be generated as follows.

Though the sensitizing antigen for generation of antibodies is not limited by the animal species from which the antibodies are obtained, it is preferably derived from a mammal from which peptides or proteins actually used in the present invention are derived, such as humans, mice, or rats. Among them, sensitizing antigens derived from humans are preferred. When, for example, a human soluble HM1.24 antigen protein is used as the sensitizing antigen, the nucleotide sequence and the amino acid sequence thereof can be obtained using the gene sequence disclosed in the present invention. Furthermore, when other peptides or proteins that are subjected to fusion to soluble HM1.24 antigen protein are used as the sensitizing antigen, the peptides and the proteins can be chemically synthesized or can be obtained using genetic engineering technology.

Peptides or proteins that are used as the sensitizing antigen may be full-length or fragments thereof. As fragments, for example, C-terminal fragments or N-terminal fragments may be mentioned. Alternatively, it is possible to use, as the sensitizing antigen, cells that express proteins, peptides, or polypeptides used as the sensitizing antigen.

The mammals to be immunized with the sensitizing antigen are not specifically limited, and they are preferably selected in consideration of their compatibility with the parent cell for use in cell fusion. They generally include rodents, lagomorphs, and primates.

Rodents include, for example, mice, rats, hamsters, and the like. Lagomorphs include, for example, rabbits. Primates include, for example, monkeys. As monkeys, catarrhines (Old-World monkeys) such as cynomolgi (crab-eating macaque), rhesus monkeys, sacred baboons, chimpanzees etc. are used.

Immunization of animals with a sensitizing antigen is carried out using a known method. A general method, for example, involves the intraperitoneal or subcutaneous administration of a sensitizing antigen to the mammal. Specifically, a sensitizing antigen which has been diluted and suspended in an appropriate amount of phosphate buffered saline (PBS) or physiological saline etc. is mixed, as desired, with an appropriate amount of a common adjuvant, for example Freund's complete adjuvant. After being emulsified, it is preferably administered to the mammal for several times every 4 to 21 days. Alternatively a suitable carrier may be used at the time of immunization of the sensitizing antigen. After such immunization, the increase in the desired antibody levels in the serum is confirmed by a conventional method.

In order to obtain polyclonal antibodies, the blood of mammal that was sensitized with an antigen is removed after the increase in desired antibody levels in the serum has been confirmed. Serum is separated from the blood. As polyclonal antibodies, serum containing the polyclonal antibodies may be used, or, as desired, the fraction containing the polyclonal antibodies may be isolated from the serum.

In order to obtain a monoclonal antibody, immune cells of mammal that was sensitized with an antigen are removed and are subjected to cell fusion after the increase in the desired antibody levels in the serum has been confirmed. At this time preferred immune cells that are subjected to cell fusion include, in particular, the spleen cells.

The mammalian myeloma cells as the other parent cells, which are subjected to cell fusion with the above-mentioned immune cells, preferably include various known cell lines such as P3 (P3X63Ag8.653) (Kearney, J. F. et al., J. Immunol. (1979) 123: 1548-1550), P3X63Ag8.U1 (Yelton, D. E., et al., Current Topics in Microbiology and Immunology (1978) 81: 1-7), NS-1 (Kohler, G. and Milstein, C., Eur. J. Immunol. (1976) 6: 511-519), MPC-11 (Margulies, D.H. et al., Cell (1976) 8: 405-415), SP2/0 (Shulman, M. et al., Nature (1978) 276: 269-270), FO (de St. Groth, S. F. and Scheidegger, D., J. Immunol. Methods (1980) 35: 1-21), S194 (Trowbridge, I. S., J. Exp. Med. (1978) 148: 313-323), R210 (Galfre, G. et al., Nature (1979) 277: 131-133) and the like.

Cell fusion between the above immune cells and the myeloma cells may be essentially conducted in accordance with a known method such as is described in Milstein et al. (Kohler, G. and Milstein, C., Methods Enzymol. (1981) 73: 3-46) and the like.

More specifically, the above cell fusion is carried out in a conventional nutrient broth in the presence of, for example, a cell fusion accelerator. As the cell fusion accelerator, for example, polyethylene glycol (PEG), Sendai virus (HVJ) and the like may be used, and, in addition, an adjuvant such as dimethyl sulfoxide etc. may be added as desired to enhance efficiency of the fusion.

The preferred ratio of the immune cells to the myeloma cells to be used is, for example, 1 to 10 times more immune cells than the myeloma cells. Examples of culture media to be used for the above cell fusion include RPMI1640 medium and MEM culture medium suitable for the growth of the above myeloma cell lines, and the conventional culture medium used for this type of cell culture, and besides a serum supplement such as fetal calf serum (FCS) may be added.

In cell fusion, predetermined amounts of the above immune cells and the myeloma cells are mixed well in the above culture liquid, to which a PEG solution previously heated to about 37°C, for example a PEG solution with a mean molecular weight of about 1000 to 6000, is added at a concentration of 30 to 60% (w/v), and mixed to obtain the desired fusion cells (hybridomas). Then by repeating the sequential addition of a suitable culture medium and centrifugation to remove the supernatant, cell fusion agents etc. which are undesirable for the growth of the hybridoma can be removed.

Said hybridoma is selected by culturing in a conventional selection medium, for example, HAT culture medium (a culture liquid containing hypoxanthine, aminopterin, and thymidine). Culturing in said HAT culture medium is continued, generally, for a period of time sufficient to effect killing of the cells other than the desired hybridoma (non-fusion cells), generally several days to several weeks. Then, the conventional limiting dilution method is conducted in which the hybridomas that produce the desired antibody are selected and cloned.

In addition to obtaining the above hybridoma by immunizing an animal other than human with an antigen, it is also possible to sensitize human lymphocytes infected with, for example, EB virus with a peptide or protein, cells expressing them, or their lysates in vitro, and to allow the resulting sensitized lymphocytes to be fused with a human-derived myeloma cell having a permanent division potential, for example U266, and thereby to obtain a hybridoma producing a desired human antibody having an activity of binding the peptide or the protein (see Japanese Unexamined Patent Publication (Kokai) No. 63-17688).

Furthermore, a transgenic animal having a repertoire of human antibody genes is immunized with an antigen peptide or protein, cells expressing them or lysates thereof to obtain the antibody-producing cells, which are used to obtain human antibody against the peptide or protein for use in the present invention using hybridomas fused to myeloma cells (see International Patent Application WO 92-03918, WO 93-2227, WO 94-02602, WO 94-25585, WO 96-33735 and WO 96-34096).

The monoclonal antibody-producing hybridomas thus constructed can be subcultured in a conventional culture medium, or can be stored for a prolonged period of time in liquid nitrogen.

In order to obtain monoclonal antibodies from said hybridoma, there may be employed a method in which said hybridoma is cultured in a conventional method and antibodies are obtained in the culture supernatant, or a method in which the hybridoma is administered to and grown in a mammal compatible with said hybridoma and antibodies are obtained in the ascites. The former method is suitable for obtaining high-purity antibodies, whereas the latter is suitable for a large scale production of antibodies.

In addition to using a hybridoma to produce antibody, immune cells that produce the desired antibody, for example the sensitized lymphocytes that have been immortalized with an oncogene, may be used to obtain antibody.

A monoclonal antibody thus produced can also be obtained as a recombinant antibody by recombinant gene technology. For example, an antibody gene may be cloned from a hybridoma or an immune cell such as sensitized lymphocytes that produce antibodies, and is integrated into a suitable vector which is then introduced into a host to produce said antibody. Recombinant antibodies may also be used in the present invention (see, for example, Borrebaeck, C.A.K., and Larrick, J. W., THERAPEUTIC MONOCLONAL ANTIBODIES, published in the United Kingdom by MACMILLAN PUBLISHERS LTD. 1990).

Antibodies for use in the present invention may be antibody fragments or modified versions thereof as long as they have the desired binding activity. For example, as fragments of antibody, there may be mentioned Fab, F(ab')₂, Fv or single-chain Fv (scFv) in which Fv or Fv's of the H chain and the L chain were ligated via a suitable linker. Specifically, antibodies are treated with an enzyme such as papain or pepsin, to produce antibody fragments, or genes encoding these antibody fragments are constructed and then introduced into an expression vector, which is expressed in a suitable host cell (see, for example, Co, M. S. et al., J. Immunol. (1994) 152, 2968-2976; Better, M. and Horwitz, A.H., Methods in Enzymology (1989) 178, 476-496; Plucktrun, A. and Skerra, A., Methods in Enzymol. (1989) 178, 497-515; Lamoyi, E., Methods in Enzymol. (1986) 121, 652-663; Rousseaux, J. et al., Methods in Enzymol. (1986) 121, 663-669; Bird, R.E. and Walker, B.W., Trends Biotechnol. (1991) 9, 132-137). Chimeric antibody or humanized antibody produced by known methods can be used in the present invention.

Antibodies produced and expressed by the immunochemical assay of the present invention may be any of the above antibodies, such as antibody produced by a hybridoma, recombinant antibody, chimeric antibody, and humanized antibody.

Antibodies that were expressed and produced as described above can be separated from the inside or outside of the host cell and then may be purified to homogeneity. Separation and purification of the antibody for use in the present invention may be accomplished by, but is not limited to, the separation and the purification methods conventionally used for protein.

These methods include chromatography columns such as affinity chromatography, filtration, ultrafiltration, salting-out, dialysis, SDS polyacrylamide gel electrophoresis, isoelectric focusing, and the like, from which methods can be selected and combined as appropriate for separation and purification of antibodies (Antibodies: A Laboratory Manual, Ed Harlow and David Lane, Cold Spring Harbor Laboratory, 1988).

As columns for use in affinity chromatography, there can be mentioned Protein A column and Protein G column. Examples of the columns used in the Protein A column are Hyper D, POROS, Sepharose F. F. (Pharmacia) and the like.

As chromatography other than the above-mentioned affinity chromatography, there can be mentioned, for example, ion exchange chromatography, hydrophobic chromatography, gel-filtration, reverse phase chromatography, adsorption chromatography, and the like (Strategies for Protein Purification and Characterization: A Laboratory Course Manual, Ed Daniel R. Marshak at al., Cold Spring Harbor Laboratory Press, 1986). These chromatographies can be carried out using a liquid chromatography such as HPLC and FPLC.

The concentration measurement and activity confirmation of the antibody obtained as above can be made by known methods such as enzyme-linked immunosorbent assay (ELISA), enzymeimmunoassay (EIA), radioimmunoassay (RIA), or fluorescent antibody assay.

Hybridoma HM1.24 that produces anti-HM1.24 antibody has been internationally deposited under the provisions of the Budapest Treaty on September 14, 1995, with the National Institute of Bioscience and Human Technology, Agency of Industrial Science and Technology, of 1-3, Higashi 1-chome, Tsukuba-shi, Ibaraki pref., Japan, as FERM BP-5233.

### Examples

The present invention will now be explained, in more detail, with reference to the examples. It should be noted, however, that the present invention is not limited to the Examples in any way.

### Example 1. Construction of a vector expressing FLAG-tagged soluble HM1.24 antigen

A HEF expression vector (International Patent Application WO 92-19759) prepared by digesting with EcoRI (manufactured by Takara Shuzo) and NotI (manufactured by Takara Shuzo) and a gene pair encoding immunoglobulin (Ig) leader sequence and a FLAG tag (manufactured by Sawady Technology) were reacted and ligated at 16°C for 3 hours in a reaction mixture containing 50 mM Tris-HCl, pH 7.6, 10 mM MgCl₂, 10 mM dithiothreitol, 1 mMTP, 50 mg/ml polyethylene glycol and 1 unit of T4 DNA ligase (manufactured by GIBCO-BRL).

The gene encoding the inserted Ig leader sequence and the FLAG tag was a synthetic gene pair shown in SEQ ID NO: 12 and 13 in which EcoRI, KpnI (manufactured by Takara Shuzo) and NotI restriction enzyme recognition sites were connected as linkers. The ligation mixture was then added to competent cells of Escherichia coli DH5a (manufactured by GIBCO-BRL), which was allowed to stand for 30 minutes on ice, for 1 minute at 42°C, and again for 1 minute on ice.

Subsequently, 400 µl of the 2xYT medium (Molecular Cloning: A Laboratory Manual, Sambrook et al., Cold Spring Harbor Laboratory Press, (1989)) was added thereto, incubated at 37°C for one hour, and then the E. coli was plated on a 2xYT agar medium (Molecular Cloning: A Laboratory Manual, Sambrook et al., Cold Spring Harbor Laboratory Press, (1989)) containing 50 µg/ml of ampicillin, and then incubated overnight at 37°C to obtain an E. coli transformant. The transformant was cultured overnight at 37°C in the 2xYT medium containing 50 µg/ml of ampicillin, and then from this culture, plasmid DNA was prepared using the alkali method (Molecular Cloning: A Laboratory Manual, Sambrook et al., Cold Spring Harbor Laboratory Press, (1989)).

On the other hand, a gene for the extracellular region of HM1.24 antigen was amplified by the PCR method using the Thermal Cycler (manufactured by Perkin Elmer Cetus). Using the cDNA of HM1.24 antigen as a template, a mixture containing 100 pmole of primers shown in SEQ ID NOs: 9 to 10, 10 mM Tris-HCl, pH 8.3, 50 mM KCl, 0.1 mM dNTPs (dATP, dGTP, dCTP, dTTP), 1.5 mM MgCl₂, and 5 units of DNA polymerase Ampli Taq (manufactured by Perkin Elmer Cetus) was first denatured at 94°C, and then was subjected to 30 cycles of 94°C for 1 minute, 55°C for 1 minute, and 72°C for 1 minute, and finally incubated at 72°C for 10 minutes.

The PCR product as the gene in the extracellular region of HM1.24 antigen was reacted and ligated to the above plasmid DNA digested with KpnI and BamHI in a reaction mixture containing 50 mM Tris-HCl, pH 7.6, 10 mM MgCl₂, 10 mM dithiothreitol, 1 mM ATP, 50 mg/ml polyethylene glycol and one unit of T4 DNA ligase (manufactured by GIBCO-BRL) at 16°C for 3 hours. Similarly to the above, the ligation mixture was added to the competent cells of Escherichia coli DH5a, and an E. coli transformant was obtained, from which plasmid DNA was prepared.

This plasmid DNA was used as a FLAG-tagged soluble antigen expression plasmid and was designated as pSFHM1.24. The sequencing of the nucleotide sequence thereof was carried out using an automatic DNA sequencer (manufactured by Applied Biosystem Inc.) and the Taq Dye Terminator Cycle Sequencing Kit (manufactured by Applied Biosystem Inc.) according to the protocol indicated by the manufacturer. As a result, a structure was confirmed that permits the expression of a fusion protein (SEQ ID NO: 2) in which the soluble antigen is connected to the tag peptide of FLAG.

### Example 2. Construction of HA-tagged soluble antigen expression plasmid

Using FLAG-tagged soluble antigen expression plasmid, HA-tagged soluble antigen expression plasmid was constructed.

First, a gene encoding the epitope of hemagglutinin was inserted to a Bluescript SK-vector (Molecular Cloning: A Laboratory Manual, Sambrook et al., Cold Spring Harbor Laboratory Press, (1989)) containing the cytomegalovirus (CMV) promoter/enhancer, the neomycin resistant gene, the dihydrofolate reductase (DHFR) gene, and a leader sequence.

The gene encoding the epitope of hemagglutinin (amino acid sequence: YPYDVPDYA) used was a synthetic DNA pair (manufactured by Scimedia) in which DraIII and KpnI restriction enzyme recognition sites were connected as linkers (SEQ ID NO: 14 and 15).

Then, one µl of the ligation mixture was added to 100 µl of competent cells of E. coli JM109 (manufactured by Nippon Gene), which was then allowed to stand on ice for 30 minutes, at 42°C for 1 minute, and again on ice for 1 minute. Subsequently, 400 µl of the SOC medium (Molecular Cloning: A Laboratory Manual, Sambrook et al., Cold Spring Harbor Laboratory Press, (1989)) was added, incubated at 37°C for one hour, and then E. coli was plated on the 2xYT agar medium (Molecular Cloning: A Laboratory Manual, Sambrook et al., Cold Spring Harbor Laboratory Press, (1989)) containing 50 µg/ml of ampicillin, and then incubated overnight at 37°C to obtain an E. coli transformant.

Twenty µl of a mixture containing 10 mM Tris-HCl, pH 8.3, 50 mM KCl, 0.1 mM dNTPs, 100 pmole each of the primers shown in SEQ ID NO: 7-8, 5 units of Ampli Taq enzyme, and the above transformant as a template was subjected to 25 cycles of incubation at 94°C for 30 seconds, 55°C for 30 seconds, and 72°C for 30 seconds. An E. coli transformant whose PCR product is 220 bp was selected by a 4% agarose gel electrophoresis. The E. coli transformant was cultured overnight at 37°C in 300 ml of a LB medium (Molecular Cloning: A Laboratory Manual, Sambrook et al., Cold Spring Harbor Laboratory Press, (1989)) containing 100 µg/ml of ampicillin. From this culture, plasmid DNA was prepared by the alkali method (Molecular Cloning: A Laboratory Manual, Sambrook et al., Cold Spring Harbor Laboratory Press, (1989)). The plasmid containing the gene encoding the epitope tag of hemagglutinin thus prepared was designated as CGM/HA.

On the other hand, the soluble HM1.24 antigen gene (sHM) used was obtained from pSFHM1.24. The soluble antigen was prepared from 5 µg of pSFHM1.24 and BamHI-digested reaction mixture by purifying a 410 bp fragment using 1% agarose gel.

Subsequently, sHM was inserted into CGM/HA. After digesting CGM/HA with KpnI and BamHI, it was reacted in a reaction mixture containing 50 mM Tris-HCl, pH 9.0, 1 mM MgCl₂, and 2 units of alkaline phosphatase (E. coli C75)(manufactured by Takara Shuzo) at 65°C for 15 minutes and was dephosphorylated. 100 ng of the dephosphorylated CGM/HA and sHM were reacted in a reaction mixture containing 5 µl of DNA ligation kit Ver. 2 (manufactured by Takara Shuzo) I solution at 16°C for 1 hour and were ligated.

Then, 1 µl of the ligation mixture was added to 100 µl of the competent cells of E. coli JM109 (manufactured by Nippon Gene), which was then allowed to stand on ice for 30 minutes, at 42°C for 1 minute, and again on ice for 1 minute. Subsequently, 400 µl of the SOC medium (Molecular Cloning: A Laboratory Manual, Sambrook et al., Cold Spring Harbor Laboratory Press, (1989)) was added, incubated at 37°C for one hour, and then the E. coli was plated on the 2xYT agar medium (Molecular Cloning: A Laboratory Manual, Sambrook et al., Cold Spring Harbor Laboratory Press, (1989)) containing 50 µg/ml of ampicillin, and then incubated overnight at 37°C to obtain an E. coli transformant.

Twenty µl of a mixture containing 10 mM Tris-HCl, pH 8.3, 50 mM KCl, 0.1 mM dNTPs, 100 pmole each of the primers shown in SEQ ID NO: 7-8, 5 units of Ampli Tag enzyme (manufactured by Perkin Elmer Cetus), and the above transformant as a template were subjected to 25 cycles of incubation at 94°C for 30 seconds, 55°C for 30 seconds, and 72°C for 30 seconds. An E. coli transformant whose PCR product is 630 bp was selected by a 1% agarose gel electrophoresis.

The E. coli transformant was cultured overnight at 37°C in 600 ml of a LB medium (Molecular Cloning: A Laboratory Manual, Sambrook et al., Cold Spring Harbor Laboratory Press, (1989)) containing 100 µg/ml of ampicillin. From this culture, plasmid DNA was prepared by the alkali method (Molecular Cloning: A Laboratory Manual, Sambrook et al., Cold Spring Harbor Laboratory Press, (1989)). The plasmid containing the gene encoding the epitope tag of hemagglutinin thus prepared and the gene encoding soluble HM1.24 antigen was designated as CGM/HA-sHM, and was used as a HA-tagged soluble HM1.24 antigen expression plasmid.

### Example 3. The determination of nucleotide sequence

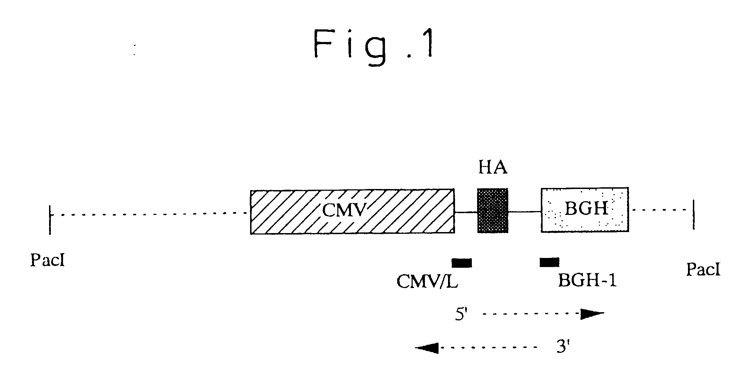

The sequencing of the nucleotide sequence of the gene encoding the epitope tag of hemagglutinin was carried out using an automatic DNA sequencer (manufactured by Applied Biosystem Inc.) and the Tag Dye terminator Cycle Sequencing Kit (manufactured by Applied Biosystem Inc.) according to the protocol indicated by the manufacturer. The primers used in the reaction are shown in SEQ ID NOs: 7 and 8, the range of the nucleotide sequence decoded in Figure 1, and the nucleotide sequence determined in SEQ ID NO: 5. This confirmed an agreement with the theoretical sequence.

### Example 4. Transfection to COS-7 cells

In order to observe the transient expression of HA-tagged soluble HM1.24 antigen, the above-mentioned expression vector was tested in COS-7 cells (ATCC #CRL-1651). The expression plasmid (CGM/HA-sHM) was co-transformed to COS-7 cells by the electroporation method using the Gene Pulser instrument (manufactured by Bio-Rad). 1 µg of the expression plasmid was added to 0.8 ml aliquots of 1 x 10⁷ cells/ml in PBS, and the mixture was subjected to pulses of 1.5 kV and 25 µFD capacity.

After a 10 minute recovery period at room temperature, the electroporated cells were cultured in a DMEM culture medium (manufactured by GIBCO-BRL) containing 10% bovine fetal serum (manufactured by GIBCO-BRL) at 37°C in a 5% CO₂ incubator. After culturing for 6 days, the culture supernatant was collected, which was then centrifuged to remove cell debris, subjected to 0.22 µm filter (manufactured by MILLIPORE), and stored at 4°C.

This was used in the initial investigation on a sandwich ELISA system.

### Example 5. Transfection to CHO cells

As a host cell, the CHO cell DG44 strain (Urlaub, G. et al., Cell (1983), 33(2) 405-412) was used. Since the DG44 strain is a DHFR deletion mutant, it exhibits an auxotrophic property for glycine, purine nucleotides, and thymidine. Thus, by transfection of the neomycin resistant gene and a DHFR-expressing plasmid, DHFR+ and neomycin resistant transformed cells can be selected using the G418-added nucleoside-deficient medium. Furthermore, cells that were selected by phased increases of the concentrations of methotrexate in the medium and that survived show an increased copy number of expression plasmids and increased an amount of the product of interest.

In order to establish a stable production system of the HA-tagged soluble antigen, the above vector (CGM/HA-sHM) that was linearized by purifying 4.7 kb fragments using 0.7% agarose gel after digestion with PacI (manufactured by New England Biolabs) was simultaneously introduced into CHO cells by the electroporation method in a similar condition to the above (the above Transfection to COS-7 cells).

The gene-transfected CHO cells were suspended in a nucleoside-free CHO-S-SFM II culture medium (manufactured by GIBCO-BRL) and then were plated on a flat-bottomed 96-well plate (manufactured by FALCON) at 100 µl/well (4 x 10⁴ cells/well). After overnight culture at 37°C in a 5% CO₂ incubator, the nucleoside-free CHO-S-SFM 11 culture medium to which 1 mg/ml G418 had been added was added at 100 µl/well, and culturing was continued. In the middle of culturing, 100 µl/well culture supernatant was extracted on day 7 and 14 after plating, and 1 mg/ml G418-added nucleoside-free CHO-S-SFM II culture medium was added at 100 µl/well to exchange buffers. On day 16 after plating, microscopic observation was carried out to select the propagated clones.

Clone #1 selected with G418 was further suspended to a nucleoside-free CHO-S-SFM II culture liquid to which 5 nM MTX (manufactured by SIGMA) had been added, and then was plated on a flat-bottomed 96-well plate at 100 µl/well in three different concentrations: 5 x 10² cells/well, 5 x 10³ cells/well, and 5 x 10⁴ cells/well. After culturing overnight at 37°C in a 5% CO₂ incubator, 100 µl/well of 5 nM MTX-added CHO-S-SFM II selection medium was added. Medium was exchanged in the middle of culturing, and microscopic observation was carried out on days 7-14. Colonies #A, #B, and #C thus obtained were subjected to an expansion culture, and, as in the case of amplification with 5 nM MTX, the concentration was increased to 50 nM and to 500 nM for amplification. Finally, the CHO cells that produce 60% of the amount in the culture supernatant by COS-7 cells were obtained.

### Example 6. Preparation of soluble antigen

In order to secure transiently soluble HM1.24 antigen, #1 strain (described in the section of Transfection to CHO cells) selected with G418 was cultured. The culture medium used was a nucleoside-free CHO-S-SFM II culture medium containing 1 mg/ml G418. It was cultured in two 500 ml spinar flasks (manufactured by Techne) (equal to 1 liter) under shaking at 60 rpm at 37°C for 10 days. It was centrifuged to collect the culture supernatant, which was passed through a 0.22 µm filter (manufactured by FALCON) and then stored at -80°C as a soluble antigen. It is believed that the HM1.24 antigen purified from the culture supernatant can be used for a search for a HM1.24 ligand, or function analysis of HM1.24 antigen.

### Example 7. Preliminary investigation of the ELISA system

The culture supernatant from COS-7 cells was used to conduct a preliminary investigation of the ELISA system. As a control for anti-HA antibody, anti-IL-6 receptor antibody, MT18 antibody (mouse IgG2bk, (Hirata, Y et al., J. Immunol. (1989) 143(9) 2900-2906)) was used. As a control for chimeric anti-HM1.24 antibody (see Reference example), myeloma-derived human IgG1 ("human IgG1 kappa purified" (manufactured by THE BINDING SITE) was purified on Protein A column similarly to chimeric antibody) was used.

### 1. Anti-HA antibody-chimeric anti-HM1.24 antibody

Anti-HA antibody (mouse monoclonal antibody; clone 12CA5, manufactured by Boehringer Mannheim) and the control antibody were prepared at 1 µg/ml and 5 µg/ml in C.B. (coating buffer: 0.1 M NaHCO₃, buffer, pH 9.6, 0.02% sodium azide), which were used to coat a flat-bottomed 96-well plate (Immuno plate I, assayed: manufactured by Nunc) at 100 µl/well at 4°C overnight. After washing three times in R.B. (rinse buffer: PBS, 0.05% Tween 20), D.B. (dilution buffer; 50 mM Tris-HCl, pH 8.1, 1 mM MgCl₂, 0.15 M NaCl, 0.05% Tween 20, 0.02% sodium azide, 1% BSA (manufactured by SIGMA)) was added at 200 µl/well for blocking at room temperature for 2 hours. Four serial dilutions of the culture supernatant in D.B. by a factor of 4 were reacted at room temperature for 1 hour.

After washing three times in R.B., chimeric anti-HM1.24 antibody diluted at 5 µg/ml in D.B. was added at 100 µl/well, and then reacted at room temperature for 1 hour. After further washing three times in R.B., alkaline phosphatase-labeled goat anti-human IgG antibody (manufactured by BIOSOURCE) diluted 5000-fold and 10,000-fold in D.B. was added at 100 µl/well and reacted at room temperature for 1 hour. After washing five times in R.B., SIGMA 104 (p-nitrophenyl phosphate, disodium hexahydrate: manufactured by SIGMA), a substrate for alkaline phosphatase, diluted at 1 mg/ml in S.B. (substrate buffer: 0.05M NaHCO₃ buffer, pH 9.8, 10 mM MgCl₂) was added at 100 µl/well, and allowed to develop color at room temperature, and then the absorbance at 405 nm -655 nm was measured by the microplate reader model 3550 (manufactured by BIORAD).

### 2. Chimeric anti-HM1.24 antibody-anti-HA antibody

Chimeric anti-HM1.24 antibody and the control antibody were prepared at 1 µg/ml and 5 µg/ml in C.B., which were used to coat a flat-bottomed 96-well plate (Immuno plate I: manufactured by Nunc) at 100 µl/well at 4°C overnight. After washing in R.B., anti-HA antibody diluted to 5 µg/ml in D.B. was added at 100 µl/well and was reacted at room temperature for 1 hour. Alkaline phosphatase-labeled goat anti-mouse IgG antibody (manufactured by ZYMED) diluted 5,000-fold and 10,000-fold was added at 100 µl/well and reacted at room temperature for 1 hour. After washing five times in R.B., SIGMA 104 was added as above for color development, and then the absorbance at 405 nm -655 nm was measured.

In the above test, a curve dependent on the concentration of antigen was drawn in either of the ELISA systems: a) anti-HA antibody → chimeric anti-HM1.24 antibody, b) chimeric anti-HM1.24 antibody → anti-HA antibody, and either could be used. Since the method of 1. is more suitable as a system for measuring humanized anti-HM1.24 antibody, the sandwich ELISA system of the above 1. was used.

### Example 8. Sandwich ELISA system that employs the culture supernatant

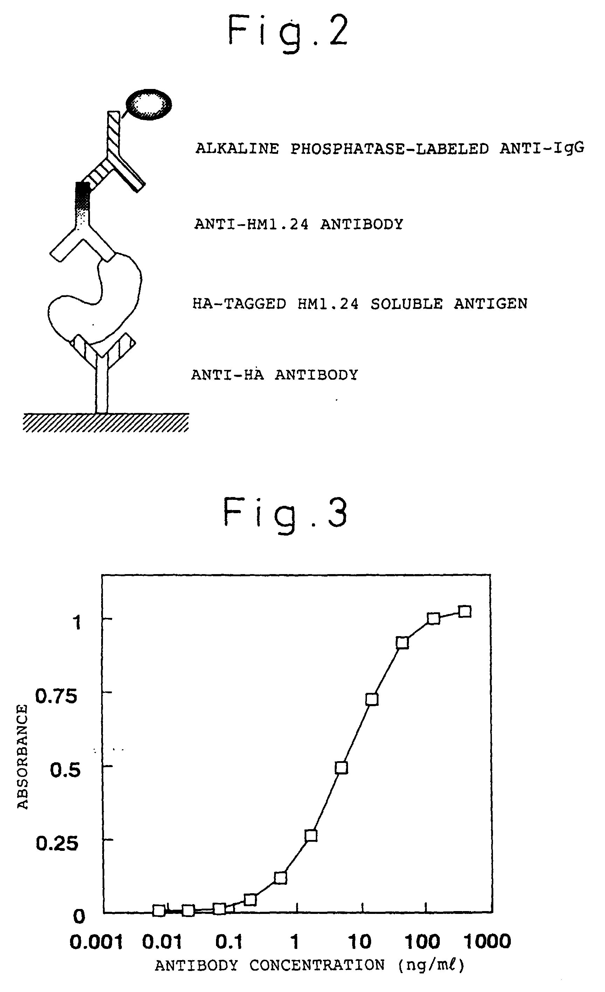

The sandwich ELISA system that employs HA-tagged soluble HM1.24 antigen is schematically shown in Figure 2.

### 1. Culture supernatant by COS-7 cells

Anti-HA antibody was coated at 1 µg/ml and 100 µl/well of the 4-fold diluted culture supernatant by COS-7 cells was added thereto, and reacted at room temperature for 2 hours. 400 ng/ml of chimeric anti-HM1.24 antibody and humanized anti-HM1.24 antibody (see Reference example) were serially diluted by a factor of three, which were added at 100 µl/well and reacted at room temperature for 1 hour. Alkaline phosphatase-labeled goat anti-human IgG (manufactured by BIOSOURCE) was added and reacted at room temperature for 1 hour. The substrate for alkaline phosphatase was similarly subjected for color development, and absorbance at 405 nm - 655 nm was measured.

As a result, a standard curve was created for humanized anti-HM1.24 antibody when the culture supernatant of COS-7 cells was used as shown in Figure 3, which showed the detection limit of the ELISA system is 500 pg/ml. The same result was obtained for chimeric antibody.

### 2. Culture supernatant by CHO cells

Using the culture supernatant (described in Preparation of soluble antigen), a sandwich ELISA was carried out in a similar manner to that described in the section of Culture supernatant by COS-7 cells. However, in order to secure the reaction of soluble antigen, reaction was carried out overnight at 4°C and AHM was subjected to a serial 3-fold dilution from 1 µg/ml.

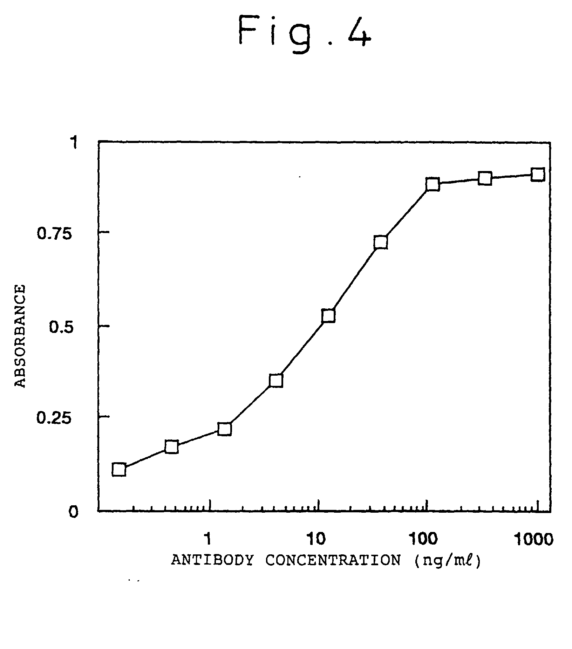

As a result, a standard curve, when soluble antigen by CHO cells was used, is as shown in Figure 4 and the detection limit thereof was about a few ng/ml.

### Example 9. Selection of cell lines

In order to obtain strains having the highest possible production ability, the amount produced of soluble antigen was compared in a sandwich ELISA with anti-HA antibody, chimeric anti-HM1.24 antibody and humanized anti-HM1.24 antibody for selection of cell lines.

After blocking the plate coated with 1 µg/ml of anti-HA antibody, serially diluted culture supernatants of HA-tagged soluble antigen-producing cells were added. Since the purified antigen was not available and thus antigen concentration was unknown, the initial amount of inoculum was rendered equal and the culture supernatant after culturing for 4 days was used.

After incubating this at room temperature for 2 hours, 1 µg/ml of chimeric anti-HM1.24 antibody and humanized anti-HM1.24 antibody were added thereto, and then incubated at room temperature for 1 hour. Alkaline phosphatase-labeled goat anti-human IgG (manufactured by BIOSOURCE) was added and reacted at room temperature for 1 hour. The substrate was added. Color was developed at room temperature, and absorbance at 405 nm - 655 nm was measured by the microplate reader model 3550 (manufactured by BIORAD).

As a result, clone #1 was selected with G418, which was then used as a parent strain and amplified with 5 nM MTX (manufactured by SIGMA) to obtain #A, #B, and #C.

### Example 10. Use of a sandwich ELISA system

### 1. Determination of blood concentrations of chimeric anti-HM1.24 antibody in Rhesus monkey that received chimeric anti-HM1.24 antibody

From a Rhesus monkey that received i.v. infusion of 4 mg/kg and 40 mg/kg of chimeric anti-HM1.24 antibody, blood was drawn on day 1, 3, 7, and 14 after the administration. The blood was centrifuged at 4°C to obtain serum. As a control group, blood was also drawn from Rhesus monkey that received physiological saline in stead of chimeric anti-HM1.24 antibody and serum was obtained. The concentration of chimeric anti-HM1.24 antibody in the serum was determined by a sandwich ELISA using HA-tagged HM1.24 soluble antigen.

A plate coated with 1 µg/ml of anti-HA antibody was blocked and 100 µl/well of HA-tagged HM1.24 soluble antigen (4-fold diluted culture supernatant by CHO cells) was added and reacted at 4°C overnight. The sera of a Rhesus monkey that received chimeric anti-HM1.24 antibody were serially diluted and 100 µl was added to each well. Chimeric anti-HM1.24 antibody that was administered as a standard was serially 3-fold diluted from 10 µg/ml by 11 times. After a 1 hour incubation at room temperature followed by washing, alkaline phosphatase-labeled goat anti-human IgG antibody (manufactured by BIOSOURCE) was added.

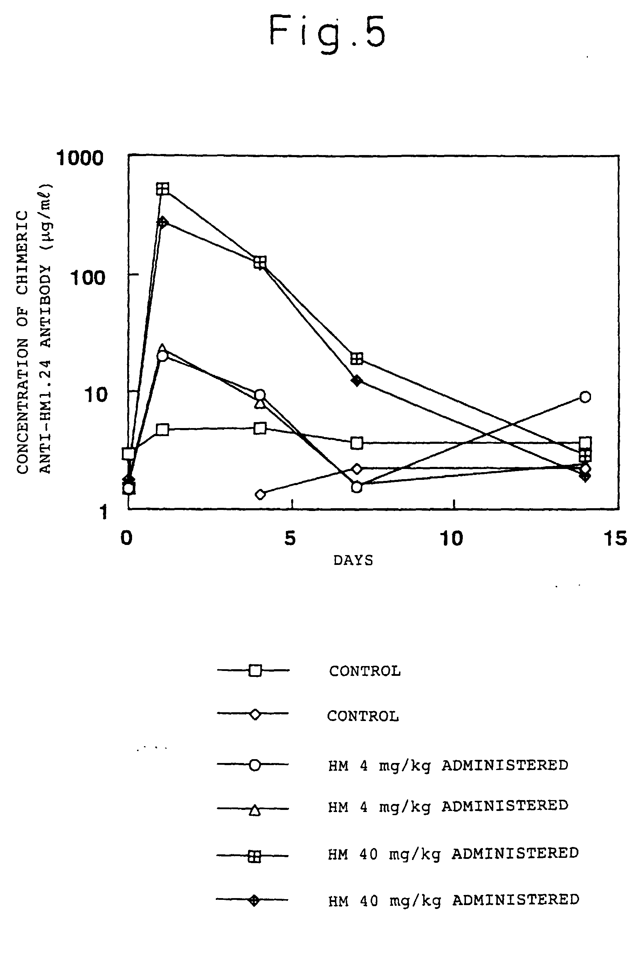

After a 1 hour incubation at room temperature followed by washing, the substrate solution was added. After incubation, absorbance at 405 nm was determined using the MICROPLATE READER Model 3550 (manufactured by BIORAD). As a result, the time course of the concentration of chimeric anti-HM1.24 antibody in the sera of Rhesus monkey was as shown in Figure 5. The detection limit of the ELISA system was 508 pg/ml.

The sandwich ELISA also permits the measurement of kinetics of mouse anti-HM1.24 antibody in the blood by replacing the second antibody with alkaline phosphatase-labeled mouse IgG2a antibody.

### 2. Measurement of the binding-inhibition activity of humanized anti-HM1.24 antibody

The binding-inhibition activity of humanized anti-HM1.24 antibody by biotin-labeled mouse anti-HM1.24 antibody was determined by a sandwich ELISA using HA-tagged HM1.24 soluble antigen. A plate coated with 1 µg/ml of anti-HA antibody was blocked, 100 µg/ml of HA-tagged HM1.24 soluble antigen (4-fold diluted culture supernatant by CHO cells) was added, and reacted at 4°C overnight. After washing, humanized anti-HM1.24 antibody and chimeric anti-HM1.24 antibody were serially 3-fold diluted from 10 µg/ml by 7 times and 50 µl thereof was added to each well At same time 50 µl of 20 ng/ml biotin-labeled mouse anti-HM1.24 antibody was added, and reacted at room temperature for 1 hour.

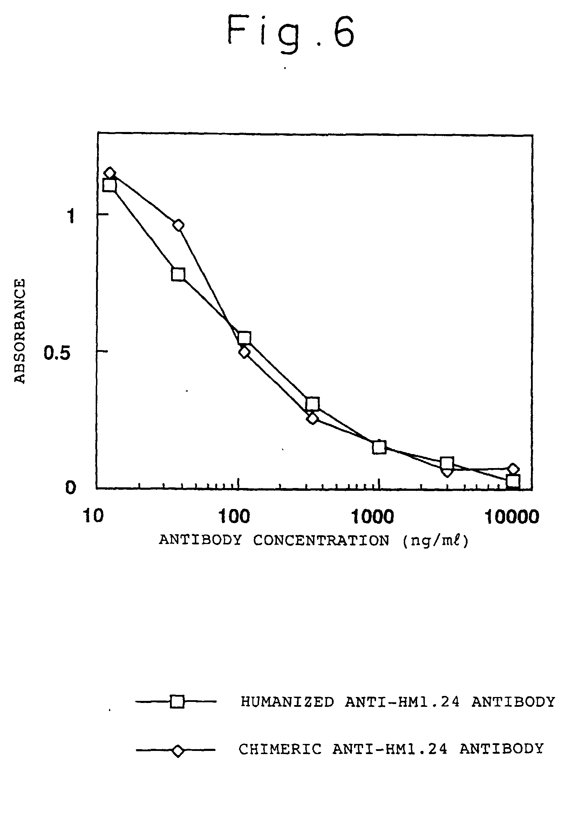

After washing, alkaline phosphatase-labeled streptavidin (manufactured by PIERCE) was added, reacted at room temperature for 2 hours followed by washing, and then the substrate solution was added. After incubation, absorbance at 405 nm was determined using the MICROPLATE READER Model 3550 (manufactured by Bio-Rad). As a result, humanized anti-HM1.24 antibody exhibited a binding-inhibition activity similar to mouse anti-HM1.24 antibody for biotin-labeled mouse anti-HM1.24 antibody as shown in Figure 6. This indicated that humanized anti-HM1.24 antibody has the same V region as mouse anti-HM1.24 antibody.

It is also applicable to an assay system of soluble HM1.24 antigen using as a standard soluble HM1.24 antigen purified from the culture supernatant.

### Example 11. FCM analysis

There was not a very high production of antigen by CHO cells that produce HA-tagged HM1.24 soluble antigen. In order to elucidate the reason for this, HA-tagged soluble antigen-producing CHO cells #1 selected with G418 and HA-tagged soluble antigen-producing CHO cells #A amplified by 5 nm MTX were analyzed by flow cytometry (FCM) analysis. After washing 1 x 10⁶ soluble antigen-producing CHO cells in PBS, 5 µl of 500 µg/ml mouse anti-HM1.24 antibody and 95 µl of the FACS buffer (PBS containing 2% bovine serum albumin and 0.1% sodium azide), or 5 µl of 500 µg/ml anti-HA antibody (manufactured by Boehringer Mannheim) and 95 µl of the FACS buffer were added, and incubated on ice for 30 minutes.

As a control, 5 µl of 500 µg/ml mouse IgG2ak (UPC10) (manufactured by CAPPEL) instead of mouse anti-HM1.24 antibody abd 95 µl of the FACS buffer, and 5 µl of mouse IgG2bk antibody (MT18) instead of mouse anti-HA antibody and 95 µl of the FACS buffer were added, and incubated similarly. After washing with the FACS buffer, 100 µl of 10 µg/ml FITC-labeled goat anti-mouse IgG antibody (manufactured by Becton Dickinson) was added, and incubated on ice for 30 minutes. After washing with the FACS buffer, the cells were suspended in 600 µl of the FACS buffer, and the fluorescence intensity of each cell was measured by the FACScan (manufactured by Becton Dickinson).

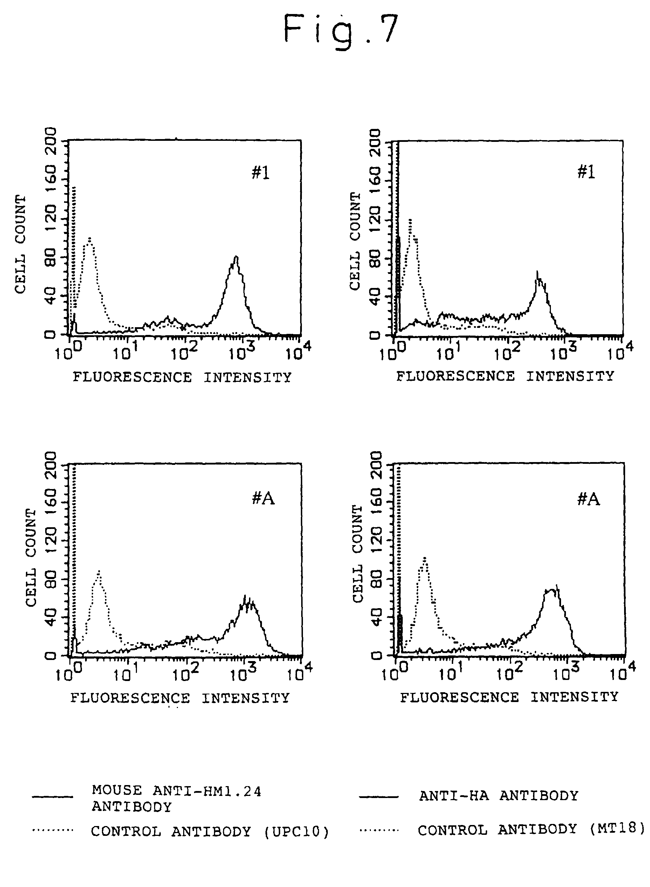

The result shown in Figure 7 revealed that, in the cells to which mouse anti-HM1.24 antibody and anti-HA antibody were added, the peak of fluorescence intensity shifted to the right compared to the control, indicating that mouse anti-HM1.24 antibody and anti-HA antibody were bound to HA-tagged soluble antigen-producing CHO cells. This confirmed that HM1.24 antigen and hemagglutinin peptide are expressed at high levels on the cell surface.

### Example 12. Generation of cell lysates

After 1.2 x 10⁷ HA-tagged soluble antigen-producing CHO cells #1 selected with G418, 1.6 x 10⁷ HA-tagged soluble antigen-producing CHO cells #A, 1.2 x 10⁷ HA-tagged soluble antigen-producing CHO cells #B, and 1.1 x 10⁷ HA-tagged soluble antigen-producing CHO cells #C amplified with 5 nM MTX were washed with PBS, 1 ml of the lysis buffer containing 50 mM NaHCO₃ buffer, pH 8.0, 150 mM NaCl, 100 µg/ml PMSF, a 25-fold diluted protease inhibitor cocktail (manufactured by Boehringer Mannheim), 0.5% sodium deoxycholate, and 1% Nonidet P-40 was added, and sonicated on ice for 30 minutes. It was then centrifuged at 14,000 rpm at 4°C for 10 minutes, and the supernatant was recovered as a cell lysate. A similar cell lysate was prepared for 1.0 x 10⁷ KPMM2 cells (Japanese Unexamined Patent Publication (Kokai) No. 7-236475) as well.

### Example 13. Western blotting

### 1. SDS polyacrylamide gel electrophoresis under a reduced condition

When preparing a cell lysate of HA-tagged soluble antigen-producing CHO cells, the culture supernatant (Sup. #1, Sup. #A, , Sup. #B, Sup. #C) obtained by culturing the cells was passed through a 0.22 µm filter and then was stored at 4°C. To 20 µl of the culture supernatant was added 10 µl of a sample buffer (0.5 M Tris-HCl buffer, pH 6.8, containing 10% glycerol, 2% SDS, 0.25% bromophenol blue) was added, and the mixture was heated to 100°C for 5 minutes. Also, to 10 µl of the cell lysate containing a cell count corresponding to 20 µl of the culture supernatant (#1: 3 x 10⁴ cells, #A: 4 x 10⁴ cells, #B: 3 x 10⁴ cells, #C: 2.8 x 10⁴ cells), 5 µl of the sample buffer containing 5% 2-mercaptoethanol was added, and the mixture was heated at 100°C for 5 minutes.

As a positive control, 5 µl of a sample buffer containing 5% 2-mercaptoethanol was added to 10 µl of the KPMM2 cell lysate that is a myeloma cell expressing HM1.24 antigen at a high level instead of a culture supernatant, which was similarly heated. Using 4-20% gradient gel (manufactured by TEFCO) SDS polyacrylamide gel electrophoresis was carried out at 20 mA for 1.5 hours. After the electrophoresis, the gel was transblotted to a PVDF membrane (manufactured by TEFCO) at 150 mA for 2 hours. The membrane was incubated overnight at 4°C in TBS-T (Tris buffer containing 0.1% Tween 20 (manufactured by Takara Shuzo)) containing 3% skim milk.

After washing with TBS-T, PBS containing 10 µg/ml mouse anti-HM1.24 antibody, 10% FCS, and 0.025% thimerosal was added, which was then reacted under shaking at room temperature for 1 hour. After washing, 1000-fold diluted peroxidase-labeled goat anti-mouse IgG antibody (manufactured by Zymed) was reacted under shaking at room temperature for 1 hour. After washing, it was subjected to chemiluminescence using an ECL detection reagent (manufactured by Amersham) and was transferred to hyper film-ECL (manufactured by Amersham). This was developed using an automatic developer (SRX-101 manufactured by Konica) to detect HM1.24 antigen.

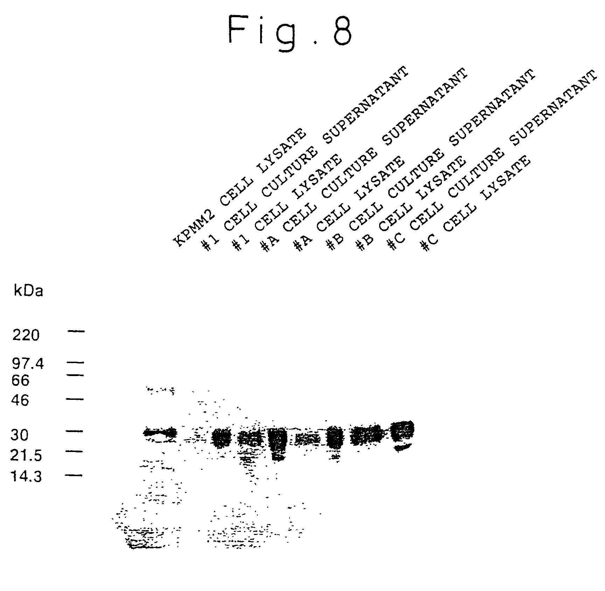

The result, as shown in Figure 8, confirmed the expression of HM1.24 antigen near 30 kDa as for the positive control KPMM2 cells.

### 2. SDS polyacrylamide gel electrophoresis under a non-reduced condition

Ten µl of the sample buffer was added to 20 µl of the above culture supernatant #C and then heated to 100°C for 5 minutes. Also, 5 µl of the sample buffer was added to 10 µl of the cell lysate #C containing 2.8 x 10⁴ cells and then heated to 100°C for 5 minutes. As a positive control, 10 µl of the sample butter was added to 10 µl of the KPMM2 cell lysate (1 x 10⁵) that is a myeloma cell expressing HM1.24 antigen at a high level instead of a culture supernatant, which was similarly heated. They were tested under the conditions described in the reduced condition to detect the antigen.

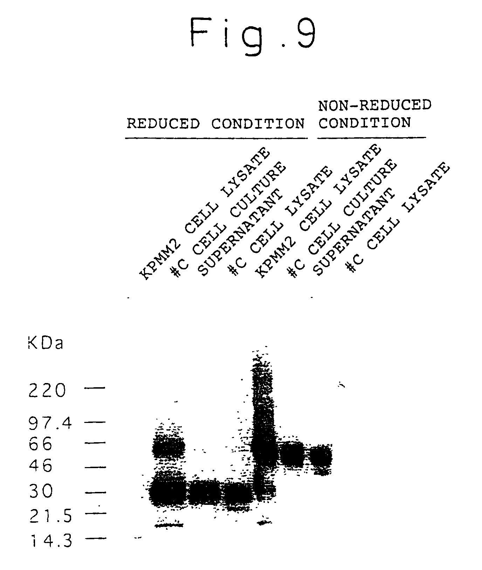

The result, as shown in Figure 9, showed that the antigen that was detected near 30 kDa under the reduced condition was detected near 60 kDa under the non-reduced condition. Thus, HA-tagged HM1.24 soluble antigen also formed a homodimer as did HM1.24 antigen.

On the other hand, since HM1.24 antigen has a hydrophobic region of about 14 amino acids in the C-terminal end, it was thought that part of the expressed antigen remained on the cell surface instead of being secreted into the culture supernatant. Thus, a HA-tagged soluble antigen in which the C-terminal containing the hydrophobic region on the C-terminal end has further been deleted from the HA-tagged soluble antigen in which the N-terminal containing the transmembrane region on the N-terminal end has further been deleted was prepared.

### Example 14. Construction of expression plasmid

A mixture containing 10 mM Tris-HCl, pH 8.3, 50 mM KCl, 0.1 mM dNTPs, 1.5 mM MgCl₂, 100 ng of pSFHM1.24 as a template, 100 pmole of each primer (SEQ ID NO: 9 and 11), and 5 units of Ampli Taq (manufactured by Perkin Elmer Cetus) was first subjected to a denaturation at 94°C, and then to 25 cycles of 94°C for 1 minute, 55°C for 1 minute, and 72°C for 1 minute, and finally incubated at 72°C for 7 minutes. The PCR product digested with KpnI and BamHI was reacted as a C-terminal deleted soluble antigen (HM164) in a reaction mixture containing 5 µg of a vector (CGM/HA), 50 mM Tris-HCl, pH 7.6, 10 mM MgCl₂, 10 mM dithiothreitol, 1 mM ATP, 50 mg/ml of polyethylene glycol and one unit of T4 DNA ligase (manufactured by GIBCO-BRL) at 16°C for 3 hours, and ligated.

Then 10 µl of the ligation mixture was added to competent cells of E. coli JM109 (manufactured by Toyobo), which was allowed to stand for 30 minutes on ice, for 1 minute at 42°C, and again for 1 minute on ice. Subsequently, 400 µl of the SOC medium (Molecular Cloning: A Laboratory Manual, Sambrook et al., Cold Spring Harbor Laboratory Press, (1989)) was added thereto, incubated at 37°C for one hour, and then the E. coli was plated on the LB agar medium (Molecular Cloning: A Laboratory Manual, Sambrook et al., Cold Spring Harbor Laboratory Press, (1989)) containing 50 µg/ml of ampicillin, and then incubated overnight at 37°C to obtain an E. coli transformant.

The transformant was cultured overnight at 37°C in the LB medium (Molecular Cloning: A Laboratory Manual, Sambrook et al., Cold Spring Harbor Laboratory Press, (1989)) containing 50 µg/ml of ampicillin, and then from this culture plasmid, DNA was prepared using the alkali method. After the plasmid DNA was digested with KpnI and BamHI, 360 bp plasmid was selected on a 1% agarose gel electrophoresis, and the selected transformant was cultured overnight at 37°C in 30 ml of the LB medium containing 50 µg/ml of ampicillin. From this culture, plasmid DNA was prepared using the alkali method (Molecular Cloning: A Laboratory Manual, Sambrook et al., Cold Spring Harbor Laboratory Press, (1989)), which was designated as (CGM/HA-HM164) as an expression plasmid of HA-tagged soluble antigen in which the hydrophobic region in the C-terminal end has been truncated.

### Example 15. Determination of the nucleotide sequence of CGM/HA-sHM164

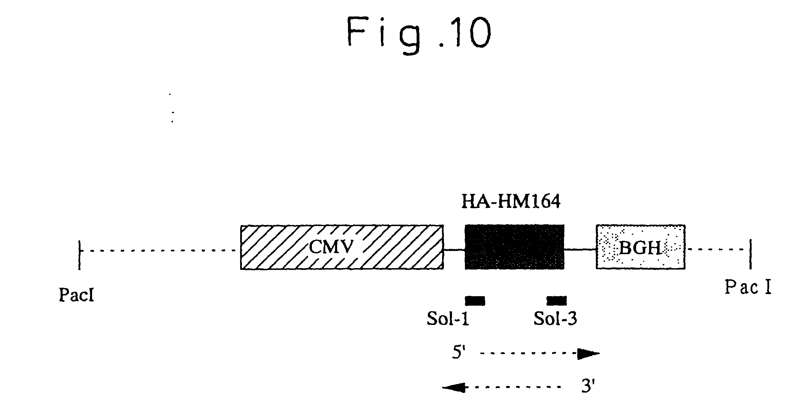

The nucleotide sequence of the expression plasmid (CGM/HA-HM164) of HA-tagged soluble antigen in which the hydrophobic region in the C-terminal end has been deleted was determined by an antomatic DNA sequencer (manufactured by Applied Biosystem Inc.) and Taq Dye terminator Cycle Sequencing kit (manufactured by Applied Biosystem Inc.) using the above expression plasmid digested with BglII (manufactured by Takara Shuzo) as a template according to the manufacturer's protocol. The primers used are shown in SEQ ID NO: 9 and 11, the range of the decoded nucleotide sequence in Figure 10, and the nucleotide sequence determined in SEQ ID NO: 6. They were confirmed to be consistent with the theoretical nucleotide sequences.

### Example 16. Transfection to COS-7 cells and CHO cells

In order to observe the transient expression of HA-tagged C-terminal deleted soluble HM1.24 antigen, the above expression vector CGM/HA-HM164 was transfected to COS-7 cells by the electroporation method in a similar manner to the one described above (the above Working Example 4. Transfection to COS-7 cells). After culturing the electroporated cells for 6 days in a similar manner to the one described above (the above Working Example 4), the recovered culture supernatant was subjected to 0.22 µm filter (manufactured by MILLIPORE), and stored at 4°C.

In order to establish a stable production system of HA-tagged soluble HM1.24 antigen, it was digested with PacI (manufactured by New England Biolabs), and then the above vector (CGM/HA-HM164) that was linearized by purifying 4.7 kb fragments using 0.7% agarose gel after digestion was simultaneously transfected into CHO cells strain DG44 (Urlaub, G. et al., Cell (1983) 33(2) 405-412) by the electroporation method in a similar condition to the above (the above Example 4. Transfection to COS-7 cells).

Furthermore, it was selected with G418 as in the above (the above Example 5).

### Example 17. Sandwich ELISA using a culture supernatant

This was carried out in a similar condition to that mentioned above (Working Example 7. Sandwich ELISA system that employs the culture supernatant). A plate on which anti-HA antibody (manufactured by Boehringer Mannheim) was coated at 1 µg/ml was blocked at room temperature for 2 hours. Then 100 µl/well of the 4-fold diluted culture supernatant by COS-7 cells and CHO cells was added thereto, and reacted overnight at 4°C. After washing, 1 µg/ml of humanized anti-HM1.24 antibody was serially three-fold diluted, which was added at 100 µl/well and reacted at room temperature for 1 hour. After washing, alkaline phosphatase-labeled goat anti-human IgG (manufactured by BIOSOURCE) was added and reacted at room temperature for 1 hour. After washing, the substrate solution was added, and absorbance at 405 nm - 655 nm was measured using the Microplate reader (manufactured by BIORAD).

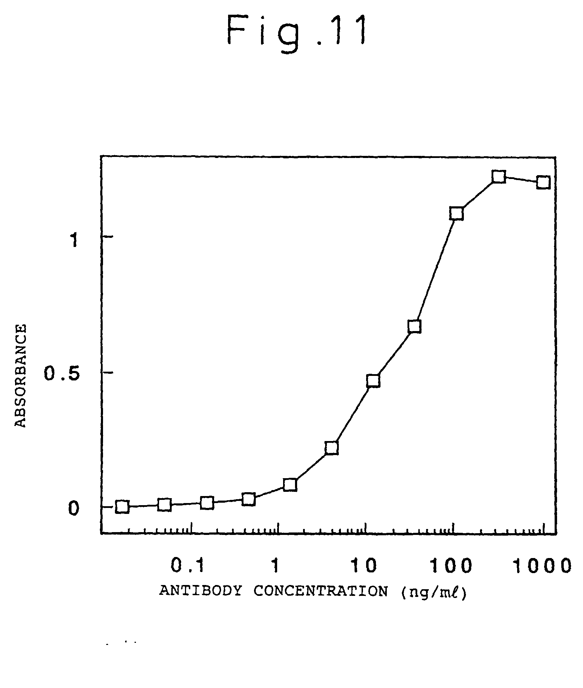

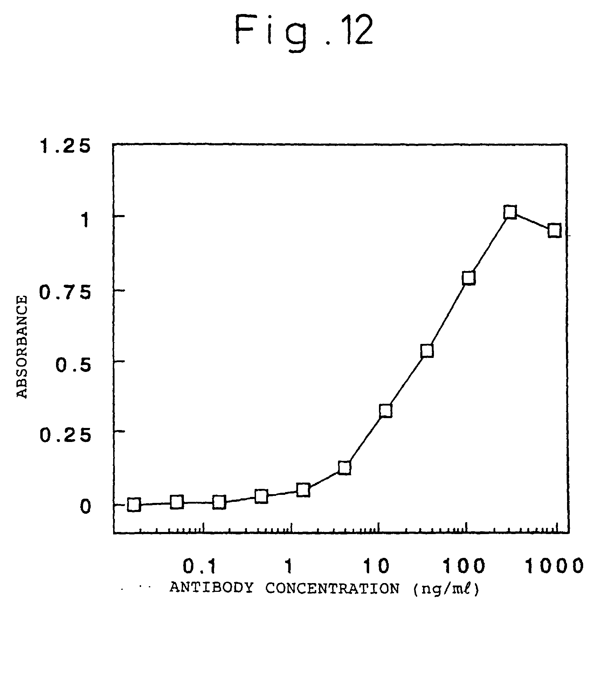

As a result, a standard curve was obtained for humanized anti-HM1.24 antibody using the culture supernatant of COS-7 cells that was allowed to produce a C-terminal deleted soluble antigen as shown in Figure 11. A standard curve was also obtained for humanized anti-HM1.24 antibody when the culture supernatant of CHO cells was used as shown in Figure 12. The detection limit for either of the ELISA systems was a few ng/ml.

### Example 18. Selection of cell lines

In order to select strains having a high production ability, the amounts of soluble antigen produced by anti-HA antibody and chimeric anti-HM1.24 antibody were compared by a sandwich ELISA and cell lines were selected. After blocking a plate to which anti-HA antibody was coated at 1 µg/ml, serial dilutions of culture supernatants of HA-tagged soluble antigen-producing cells were added. Since the purified antigen was not available and thus the antigen concentration was unknown, the initial amount of inoculum was rendered equal and the culture supernatant that was cultured for 4 days was used. After incubating this at room temperature for 2 hours, 1 µg/ml of chimeric anti-HM1.24 antibody and humanized anti-HM1.24 antibody was added thereto, and then incubated at room temperature for 1 hour. After alkaline phosphatase-labeled goat anti-human IgG (manufactured by BIOSOURCE) was added and reacted at room temperature for 1 hour, the substrate was added. Color was developed at room temperature, and absorbance at 405 nm - 655 nm was measured by a microplate reader (manufactured by BIORAD).

As a result, clones #1, #2, #21, and #28 that had a high producing ability were selected in the selection of cells with G418.

### Example 19. Western blotting

First, a cell lysate was prepared. The cell lysate was prepared from 1 x 10⁷ COS-7 cells in which HA-tagged, C-terminal deleted soluble antigen was expressed, and each of 4 clones of HA-tagged, C-terminal deleted soluble antigen-producing CHO cells (#1: 1.2 x 10⁷ cells, #2: 1.5 x 10⁷ cells, #21: 2.2 x 10⁷ cells, #28: 1.3 x 10⁷ cells) was prepared under the condition of the above Working Example 12.

When preparing the cell lysate, the supernatant of the culture in which cells were cultured (COS-7 Sup., CHO Sup. #2, #21, #28) was passed through a 0.22 µm filter and then was stored at 4°C. To 20 µl of the culture supernatant was added 10 µl of a sample buffer (0.5 M Tris-HCl buffer, pH 6.8, containing 10% glycerol, 2% SDS, 0.25% bromophenol blue) containing 5% 2-mercaptoethanol was added, and the mixture was heated to 100°C for 5 minutes. Also to 10 µl of the cell lysate containing a cell count 10-16 times that corresponding to 20 µl of the culture supernatant (COS-7: 1 x 10⁵ cells, #2: 5.4 x 10⁴ cells, #21: 1 x 10⁵ cells, #28: 5.9 x 10⁴ cells), 5 µl of the sample buffer containing 5% 2-mercaptoethanol was added, and the mixture was similarly heated.

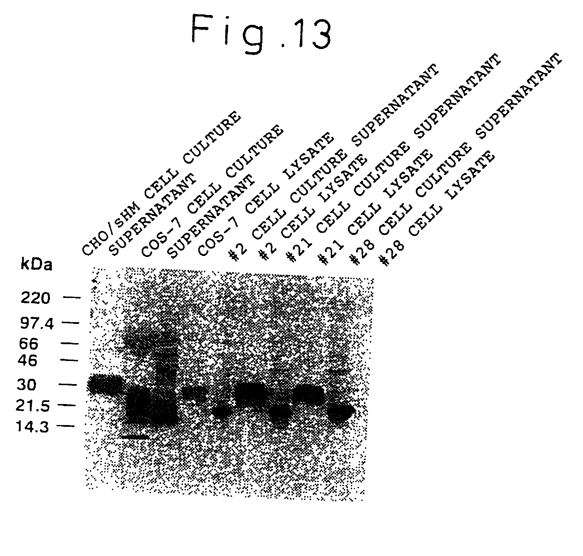

As a positive control, 20 µl of the culture supernatant (#C Sup.) of HA-tagged soluble antigen-expressing CHO cells #C from which C-terminal has not been deleted was similarly treated with 10 µl of the sample buffer containing 5% 2-mercaptoethanol. They were subjected to Western blot using mouse anti-HM1.24 antibody under the condition mentioned above (Working Example 13) and the result is shown in Figure 13. As can be seen from the figure, soluble HM1.24 antigen was detected as before, but the expression of HM1.24 antigen was not observed in the cell lysate of the C-terminal deleted soluble antigen-producing cells. Therefore, by deleting the hydrophobic region of the C-terminal end, HA-tagged HM1.24 soluble antigen that had been trapped on the cell surface came to be secreted into the culture supernatant.

Furthermore, after SDS polyacrylamide gel electrophoresis under a non-reduced condition using the culture supernatant of the above C-terminal deleted soluble antigen-producing cells and Western blot, the C-terminal deleted soluble antigen also formed a homodimer.

### Example 20.

ELISA was investigated using a GST-tagged soluble HM1.24 antigen.

### 1. Preparation of GST-tagged soluble HM1.24 antigen

The extracellular domain amino acids 76-180 (IS-1) of HM1.24 antigen was linked to the C-terminal of glutathione S-transferase (GST) to construct an expression vector of GST. IS-1, which was used to transform E. coli (DH5α) (GST. IS-1/E. coli). The GST. IS-1/E. coli was cultured overnight in the LB/Amp medium. The preculture was diluted 50-fold with the LB/Amp medium and then cultured at 30°C for 4 hours. When the density reached 0.7 or higher, IPTG was added to a concentration of 0.5 mmol/L to induce expression for about 4 hours. The E. coli cells were harvested, suspended in D-PBS (-), and lyophilized at -80°C for the following purification.

The expressed GST. IS-1 was extracted from the inclusion body of E. coli. Thus, after thawing the lyophilized E. coli, Triton X100 was added thereto to 1%, which was then crushed by sonication at a condition of output 2 and duty 50% for 1 minute. Using a Tomy MX160 it was centrifuged at 14,000 rpm for 20 minutes and the precipitated fraction was collected. The precipitate fraction was suspended in 50 mmol/L Tris-HCl, pH 8.0, containing 100 µgml Lysozyme, and was then digested on ice for 30 minutes. After digesition with Lysozyme, MgCl₂ was added to 5 mmol/L and then it was digested with DNase I at room temperature for 10 minutes. A precipitate after centrifugation at 14,000 rpm for 20 minutes was collected, and washed twice with 50 mmol/L Tris-HCl, pH 8.0, containing 1% Triton X100. To the precipitate fraction was added 50 mmol/L Tris-HCl, pH 8.0, containing 8 mol/L urea and 10 mmol/L DTT, which was suspended by pipetting. After centrifugation at 14,000 rpm for 20 minutes, the supernatant was used as a GST. IS-1 extract.

The GST. IS-1 extract was purified by anion exchange chromatography using DEAE Sepharose Fast Flow. The GST. IS-1 extract was applied to a DEAE Sepharose Fast Flow column that had been equilibrated with 50 mmol/L Tris-HCl, pH 8.0, containing 8 mol/L urea and 10 mmol/L DTT, and then washed in the same buffer. The GST. IS-1 that was adsorbed was eluted by rendering the concentration of NaCl to 0.25 mol/L and the fraction was used as GST. IS-1 (D).

Subsequently, GST. IS-1 (D) was purified by a reverse-phase HPLC using COSMOSIL C4. GST. IS-1 (D) was first diluted with 2 volumes of 200 mmol/L sodium acetate-HCl buffer, pH 3.5, and then desalted with a column of PD-10 that had been equilibrated with 20 mmol/L sodium acetate-HCl buffer, pH 3.5. To the desalted GST. IS-1 (D), an equal amount of 0.1% TFA was added, which was then allowed to adsorb to the COSMOSIL C4 that had been equilibrated with 20% CH₃CN/0.1% TFA. The adsorbed GST. IS-1 was eluted by linearly increasing the concentration of acetonitrile to 60%. The major GST. IS-1 elution fraction was designated as GST. IS-1 (C4).

Then, GSP. IS-1 (C4) was purified by a second reverse-phase HPLC using Vydac Diphenyl. GST. IS-1 (C4) was diluted 3-fold with deionized water and was allowed to adsorb to the Vydac Diphenyl that had been equilibrated with 30% CH₃CN/0.1% TFA. The adsorbed GST. IS-1 was eluted by linearly increasing the concentration of acetonitrile to 60%. GST. IS-1 was eluted as a main peak.

### 2. ELISA

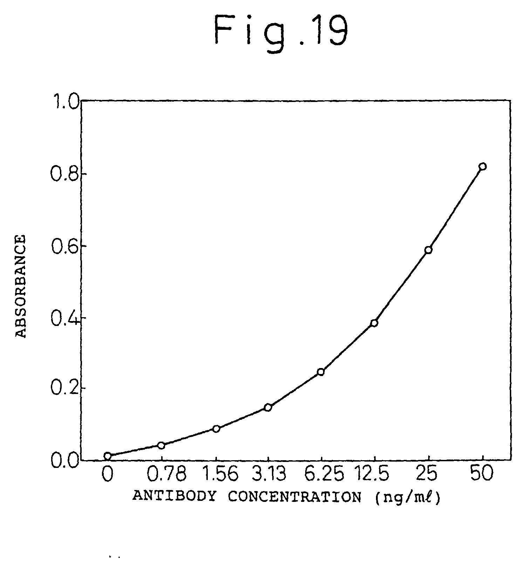

The Coating Buffer (CB) used was a 100 mmol/L NaHCO₃ solution containing 0.02% NaN₃, the dilution Buffer (DB) used was a 50 mmol/L Tris-HCl, pH 8.1, solution containing 1 mmol/L MgCl₂, 150 mmol/L NaCl, 0.05% Tween 20, 0.02% NaN₃, and 1% BSA, the substrate Buffer (SB) used was 50 mmol/L NaHCO₃, pH 9.8, solution containing 10 mmol/L MgCl₂, and the 0.1% Tween 20/TBS used was TBS (Takara Code T903, Lot 291) containing 0.1% Tween 20. GST. IS-1 (D) was directly immobilized to the Nunc Immuno Plate Maxi Sorp and the concentration of humanized anti-HM1.24 antibody was determined. GST. IS-1 (D) was diluted with CB and added to the Nunc Immuno Plate Maxi Sorp at 100 µl/well and immobilized at room temperature for 1 hour. After washing three times at 200 µl/well with 0.1% Tween 20/TBS, DB was added at 200 µl/well, to block, at room temperature for more than 1 hour. As a test sample, humanized anti-HM1.24 antibody diluted with DB was reacted at 100 µl/well at room temperature for 1 hour. Then, after washing three times with 0.1% Tween 20/TBS at 200 µl/well, alkaline phosphatase-labeled goat anti-IgG antibody (Goat anti human IgGγ chain AP conjugate) (Biosource AH20305, Lot 6202) was reacted at 100 µl/well at room temperature for 1 hour. After washing three times with 0.1% Tween 20/TBS at 200 µl/well, Sigma 104 diluted prepared at 1 mg/ml with SB was added at 100 µl/well and was allowed to develop color at room temperature for 1 hour. Absorbance at 405 nm-620 nm was determined using the Bio-Rad Model 3550. As a result, an increase in absorbance was obtained, in a dose dependent manner, depending on the concentration of humanized anti-HM1.24 antibody (Figure 19).

### Reference example 1. Preparation of hybridomas that produce mouse anti-HM1.24 monoclonal antibody

In accordance with the method of Goto, T. et al., Blood (1994) 84, 1992-1930, hybridomas that produce mouse anti-HM1.24 monoclonal antibody were prepared.

A plasma cell line KPC-32 (1 x 10⁷ cells) derived from the bone marrow of a patient with human multiple myeloma (Goto, T. et al., Jpn. J. Clin. Hematol. (1991) 32, 1400) was injected twice to the abdominal cavity of a BALB/c mouse (manufactured by Charles River) every six weeks.

Three days prior to sacrificing the animal, 1.5 x 10⁶ KPC-32 cells was injected to the spleen of the mouse in order to further enhance the antibody-producing ability of the mouse (Goto, T. et al., Tokushima J. Exp. Med. (1990) 37, 89). After sacrificing the animal the spleen was extracted and the extracted organ was subjected to cell fusion with the myeloma cell SP2/0 according to the method of Groth, de St. & Schreidegger (Cancer Research (1981) 41, 3465).

By the Cell ELISA (Posner, M.R. et al., J. Immunol. Methods (1982) 48, 23) using KPC-32, the culture supernatant of the hybridoma was screened for antibody. 5 x 10⁴ KPC-32 cells were suspended in 50 ml of PBS and then were aliquoted to a 96-well plate (U-bottomed, Corning, manufactured by Iwaki), which was then air-dried at 37°C overnight. After blocking with PBS containing 1% bovine serum albumin (BSA), the culture supernatant of the hybridoma was added thereto and incubated at 4°C for 2 hours. Then, peroxidase-labeled anti-mouse IgG goat antibody (manufactured by Zymed) was reacted at 4°C for 1 hour. After washing, an o-phenylene diamine solution (manufactured by Sumitomo Bakelite) was reacted at room temperature for 30 minutes.

The reaction was stopped by adding 2 N sulfuric acid and the absorbance was measured at 492 nm using the ELISA reader (manufactured by Bio-Rad). In order to remove the hybridoma that produced antibodies against human immunoglobulin, the culture supernatant of the positive hybridoma had previously been adsorbed to human serum and the reactivity to other cell lines was screened by ELISA. Positive hybridomas were selected, and their reactivity to various cells were investigated by flow cytometry. The last selected hybridoma clone was cloned twice, which was injected to the abdominal cavity of a pristane-treated BALB/c mouse and the ascites were obtained therefrom.

Monoclonal antibodies were purified from the ascites of the mouse by ammonium sulfate precipitation and a Protein A affinity chromatography kit (Ampure PA, manufactured by Amersham). The purified antibodies were labeled with FITC using the Quick Tag FITC binding kit (manufactured by Boehringer Mannheim).

As a result, monoclonal antibodies produced by 30 hybridoma clones reacted with KPC-32 and RPMI 8226. After cloning, the reactivity of the culture supernatant of these hybridomas with other cell lines or peripheral blood mononuclear cells was investigated.

Among them, 3 clones produced monoclonal antibodies that specifically reacted with the plasma cell. From among the 3 clones, a hybridoma clone that was most useful for flow cytometry analysis and had a CDC activity to RPMI 8226 was selected and designated as HM1.24. The subclass of the monoclonal antibody produced by this hybridoma was determined by an ELISA using a subclass-specific anti-mouse rabbit antibody (manufactured by Zymed). Anti-HM1.24 antibody had a subclass of IgG2a κ. The hybridoma HM1.24 that produces anti-HM1.24 antibody was internationally deposited under the provisions of the Budapest Treaty as FERM BP-5233 on September 14, 1995 with the National Institute of Bioscience and Human Technology, Agency of Industrial Science and Technology, of 1-3, Higashi 1-chome, Tsukuba-shi, Ibaraki pref., Japan.

### Example 2. Preparation of humanized anti-HM1.24 antibody

Humanized anti-HM1.24 antibody was obtained by the following method.

From the hybridoma HM1.24 prepared in Reference example 1, total RNA was prepared by the conventional method. From this, cDNA encoding the V region of mouse antibody was synthesized and amplified by a polymerase chain reaction (PCR) method and the 5'-RACE method. A DNA fragment containing the gene encoding the mouse V region was obtained, which was ligated to each plasmid pUC cloning vector and then introduced into competent E. coli cells to obtain an E. coli transformant. The above plasmid was obtained from the transformant. The nucleotide sequence of the cDNA coding region in the plasmid was determined in the conventional method, and the complementarity determining region (CDR) of each V region was determined.

In order to construct a vector expressing chimeric anti-HM1.24 antibody, cDNA encoding the V region of each of L chain and H chain of a mouse anti-HM1.24 antibody was inserted to the HEF vector. Furthermore, in order to construct humanized anti-HM1.24 antibody, the V region CDR of a mouse anti-HM1.24 antibody was transplanted to a human antibody by the CDR transplantation method. The L chain of human antibody REI was used as the L chain of human antibody, FR1-3 of the human antibody HG3 was used for the framework region (FR) 1-3 as the H chain of human antibody, and FR4 of the human antibody JH6 was used for FR4. The amino acid in the FR of the H chain V region was replaced so that the CDR-transplanted antibody could form a suitable antigen-binding site.

In order to express the gene of the L chain and the H chain of the thus constructed humanized anti-HM1.24 antibody in a mammalian cell, each gene was separately introduced into the HEF vector to construct a vector that expresses the L chain or the H chain of the humanized anti-HM1.24 antibody, respectively.

By simultaneously introducing these two expression vectors into the CHO cells, a cell line that produces humanized anti-HM1.24 antibody was established. The antigen binding activity and the binding inhibition activity to human amniotic membrane cell line WISH of humanized anti-HM1.24 antibody obtained by culturing this cell line was investigated by the Cell ELISA. The result indicated that humanized anti-HM1.24 antibody has an antigen binding activity equal to chimeric antibody, and for the biding inhibition activity using a biotinylated mouse anti-HM1.24 antibody as well, it had an activity equal to chimeric antibody or mouse antibody.

Incidentally, E. coli having the plasmid that contains the DNA encoding the L chain V region or the H chain V region of chimeric anti-HM1.24 antibody has been internationally deposited under the provisions of the Budapest Treaty as Escherichia coli DH5α (pUC19-1.24L-gκ) and Escherichia coli DH5α (pUC19-1.24L-gγl) on August 29, 1996 with the Fermentation Research Institute, Agency of Industrial Science and Technology, of 1-3, Higashi 1-chome, Tsukuba-shi, Ibaraki pref., Japan, as FERM BP-5646 and FERM BP-5644, respectively.

Furthermore, E. coli having the plasmid that contains the DNA encoding the a version (SEQ ID NO: 17) of the L chain V region or the r version (SEQ ID NO: 18) of the H chain V region of humanized anti-HM1.24 antibody has been internationally deposited under the provisions of the Budapest Treaty as Escherichia coli DH5α (pUC19-RVLa-AHM-gκ) and Escherichia coli DH5α (pUC19-RVHr-AHM-gγl), respectively, on August 29, 1996 with the National Institute of Bioscience and Human Technology, Agency of Industrial Science and Technology, of 1-3, Higashi 1-chome, Tsukuba-shi, Ibaraki pref., Japan, as FERM BP-5645 and FERM BP-5643, respectively.

Furthermore, E. coli having the plasmid that contains the DNA encoding the s version (SEQ ID NO: 19) of the H chain V region of humanized anti-HM1.24 antibody has been internationally deposited under the provisions of the Budapest Treaty as Escherichia coli DH5α (pUC19-RVHs-AHM-gγl) on September 29, 1997 with the National Institute of Bioscience and Human Technology, Agency of Industrial Science and Technology, of 1-3, Higashi 1-chome, Tsukuba-shi, Ibaraki pref., Japan, as FERM BP-6127.

### Reference example 3. Cloning of cDNA encoding HM1.24 antigen protein

### 1) cell lines

Human multiple myeloma cell line RPMI8226 and U266 were cultured in the RPMI1640 medium (GIBCO-BRL) supplemented with 10% fetal bovine serum (FBS), and human multiple myeloma cell line KPMM2 (Japanese Unexamined Patent Publication (Kokai) No. 7-236475) was cultured in the RPMI1640 medium (GIBCO-BRL) supplemented with 20% fetal bovine serum.

### 2) construction of cDNA library

Total RNA was prepared according to the guanidine thiocyanate/cesium chloride method from 1 x 10⁸ KPMM2 cells, and mRNA was purified using the Fast Track mRNA Isolation Kit (Invitrogen). After synthesizing cDNA from 10 µg of mRNA using NotI/oligo-dT₁₈ (Time Saver cDNA Synthesis Kit; Pharmacia Biotech), an EcoRI adapter was ligated thereto. cDNA of 0.7 kbp or greater was fractionated using 1.0% low-melting point agarose gel (Sigma), and digested with NotI. It was then inserted to the EcoRI/NotI site of a pCOS1 expression vector or a λExCell vector (Pharmacia Biotech) to prepare a library (library A) for use in direct cloning (screening by panning) and a library (library B) for use in immunoscreening, respectively.

pCOS1 expression vector was constructed from HEF-PMh-gλ1 (see WO92-19759) by deleting genes contained with EcoRI and SmaI digestion, and then by ligating it to the EcoRI-NotI-BamHI Adaptor (Takara Shuzo).

### 3) Panning

Library A was introduced to COS-7 cells by the electroporation method. Thus, 20 µg of the plasmid DNA (containing 5 x 10⁵ independent clones) was mixed with 0.8 ml of cells (1x 10⁷ cells/ml in PBS), and the mixture was subjected to electroporation under a condition of 1.5 kV and 25 µFD capacity using the Gene Pulser (Bio-Rad). After allowing to stand at room temperature for 10 minutes, the cells were suspended in a DMEM (manufactured by GIBCO-BRL) containing 10% FBS, divided into four 100 mm culture dishes, and cultured at 37°C for 72 hours.

After culturing, the cells were washed with a phosphate buffer (PBS), and were scraped off by adding PBS containing 5 mM EDTA to adjust a cell suspension of 1-2 x 10⁶ cells/ml with PBS containing 5% FBS and 0.02% NaN₃. The cells were then allowed to stand on a panning plate (see below) coated with anti-HM1.24 antibody for 2 hours, and the plate was gently washed three times with 3 ml of PBS containing 5% FBS and 0.02% NaN₃. After washing, plasmid DNA was recovered from the cells bound to the plate using the Hirt's solution (Hitt J., Mol. Biol. 26: 365-369, 1983) (0.6% SDS, 10 mM EDTA). The recovered plasmid DNA was grown in E. coli and used for the following panning.

A panning plate was prepared as follows. Three milliliters of an anti-HM1.24 antibody solution (10 µg/ml in 50 mM Tris-HCl, pH 9.5) was added to a cell culture dish (Falcon) with a diameter of 60 mm and was incubated at room temperature for 2 hours. After washing three times in 0.15 M NaCl, 3 ml of PBS containing 5% FBS, 1 mM EDTA, and 0.02% NaN₃ was added to the dish. After blocking by allowing to stand at room temperature for 2 hours, the panning plate was stored at -20°C until use.

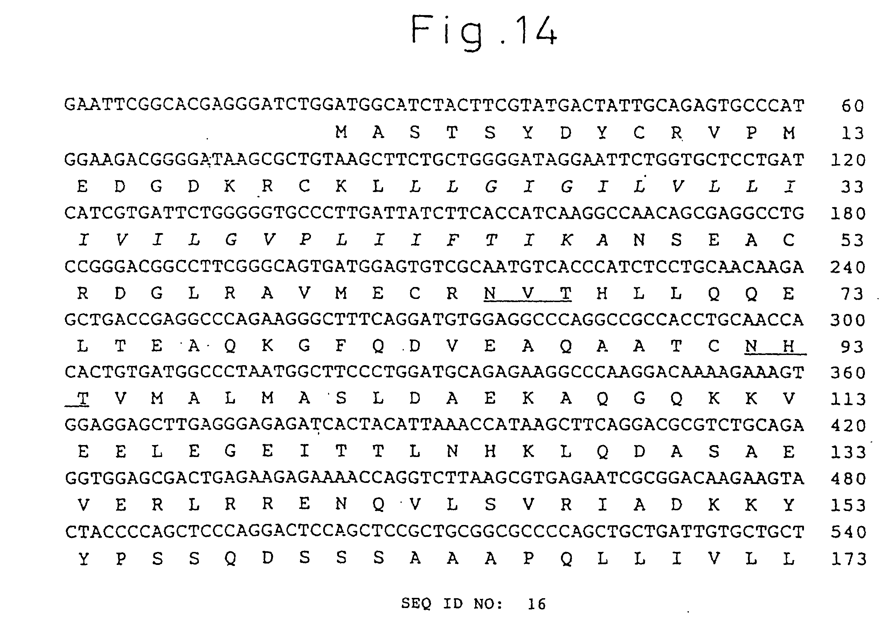

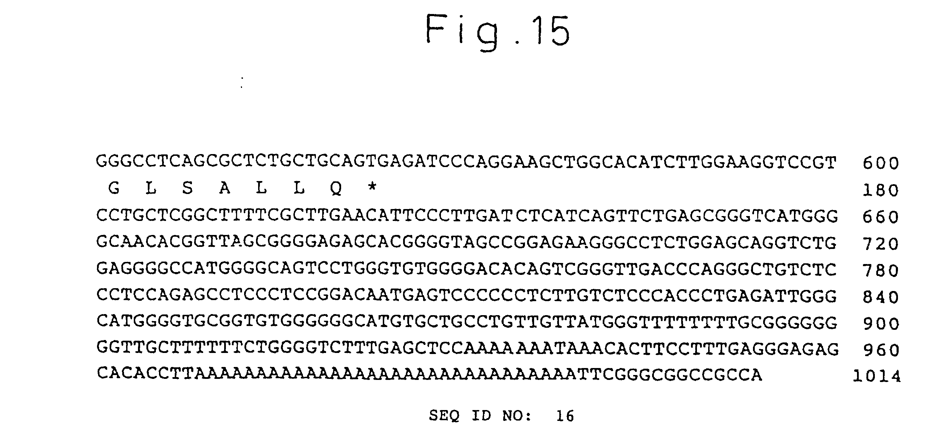

By repeating panning three times using a plasmid library (library A) containing 5 x 10⁵ clones as a starting material, a plasmid DNA having an about 0.9 kbp cDNA as an insert was concentrated. Using Dye Terminator Cycle Sequencing Kit (manufactured by Applied Biosystem Inc.), the nucleotide sequence was determined by the 373A or 377DNA Sequencer (Applied Biosystems). The result revealed that clone P3.19 comprises 1,012 bp cDNA and has an open reading frame (23-549) encoding 180 amino acids (Figures 14 and 15) (SEQ ID NO: 16). The amino acid sequence deduced from the cDNA had a structure characteristic to type II membrane proteins and had 2 N-type sugar chain binding sites.

### 4) Immunoscreening

Library B was subjected to immunoscreening using anti-HM1.24 antibody. Thus, a phage library containing 1.5 x 10⁵ independent clones was layered on agar together with E. coli NM522 (Pharmacia Biotech) and was cultured at 42°C for 3.5 hours. After culturing, a nitorocellulose filter pretreated with 10 mM IPTG was superimposed on the plate, and was further cultured at 37°C for 3 hours. After the filter was washed with 0.05% (v/v) Tween 20-added TBS (20 mM Tris-HCl, pH 7.4, 150 mM NaCl), 1% (w/v) BSA-added TBS was added thereto, and was blocked by incubating at room temperature for 1 hour.

After blocking, a anti-HM1.24 antibody solution (a 10 µg/ml blocking buffer) was added, incubated at room temperature for 1 hour, and 5,000-fold diluted alkaline phosphatase-conjugated anti-mouse Ig antiserum (picoBlue Immunoscreening kit; Stratagene) was added, which was further incubated at room temperature for 1 hour. Spots that reacted with the antibody were allowed to develop color with a developing solution (100 mM Tris-HCl, pH 9.5, 100 mM NaCl, 5 mM MgCl₂) containing 0.3 mg/ml nitroblue tetrazolium and 0.15 mg/ml 5-bromo-4-chloro-3-indolyl phosphate.

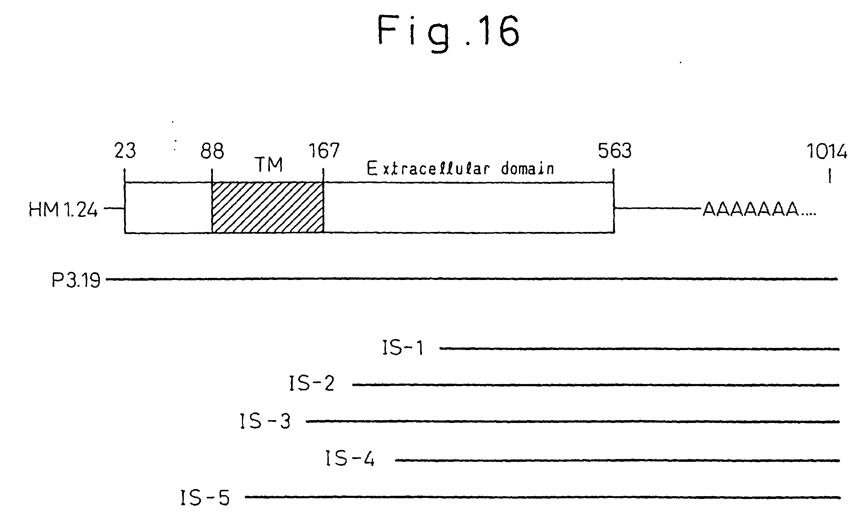

By immunoscreening, five positive clones were isolated, all of which were consistent with the partial sequence of P3.19 (Figure 16). Homology search of them revealed that P3.19 is identical with the nucleotide sequence of BST-2 (Ishikawa J. et al., Genomics, 26: 527-534, 1995) expressed on the bone marrow or synovial stromal cells. The same molecule was obtained from two runs of immunoscreening, which strongly suggested that membrane protein encoded by P3.19 is the HM1.24 antigen molecule.

E. coli having the plasmid pRS38-pUC19 in which DNA encoding human protein having the same sequence as the above-mentioned human HM1.24 antigen protein has been inserted in between the XbaI sites of pUC vector has been internationally deposited under the provisions of the Budapest Treaty as Escherichia coli DH5α (pRS38-pUC19) on October 5, 1993 with the National Institute of Bioscience and Human Technology, Agency of Industrial Science and Technology, of 1-3, Higashi 1-chome, Tsukubashi, Ibaraki pref., Japan, as FERM BP-4434.

### 5) FACS analysis

Furthermore, in order to confirm that the protein encoded by p3.19 indeed binds to anti-HM1.24 antibody, a CHO transformant cell line in which P3.19 has bee introduced was established. Thus, after P3.19 clone was introduced into CHO cells by the electroporation method, it was cultured in the presence of 500 µg/ml G418 (GIBCO-BRL) to obtain a CHO cell line that expresses HM1.24 antigen.

The cultured cells (1 x 10⁶) were suspended to the FACS buffer (PBS (-) / 2% FCS / 0.1% NaN₃), HM1.24 antibody was added thereto, which was reacted on ice for 30 minutes. After washing twice in the FACS buffer, it was resuspended in a GAM-FITC solution (25 µg/ml in the FACS buffer; Becton Dickinson), and was further reacted on ice for 30 minutes. After washing twice in the FACS buffer, it was resuspended in 600 µl of the FACS buffer for measurement by the FACScan (Becton Dickinson).

As a negative control, UPC10 was used.

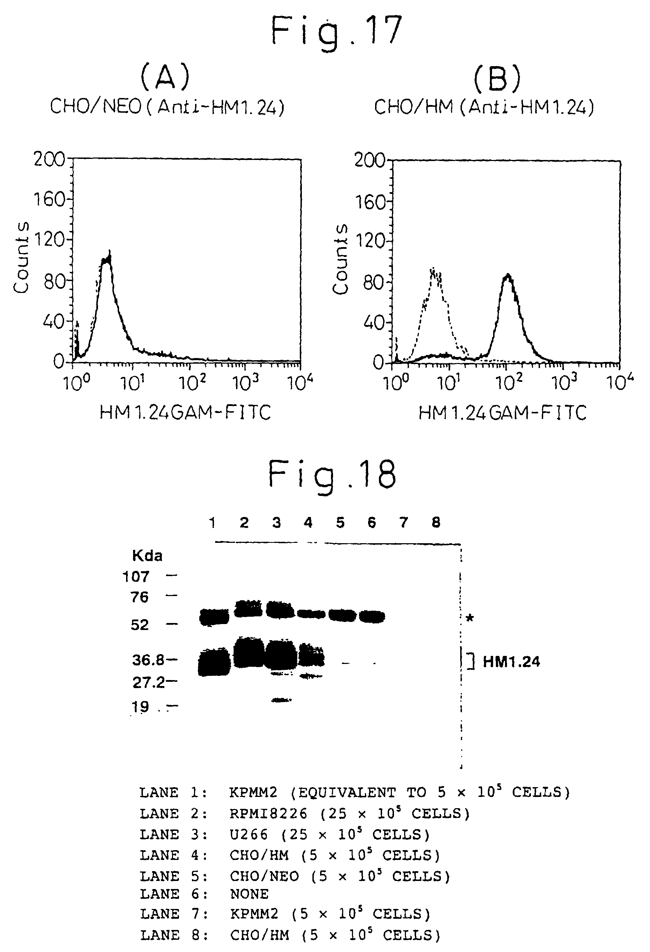

As result of FACS analysis, CHO cells in which P3.19 has been introduced were shown to react strongly with anti-HM1.24 antibody, whereas no binding was obtained in CHO cells (CHO/NEO) in which the control expression vector has only been introduced (Figure 17). It was confirmed therefore that the protein encoded by P3.19 binds to anti-HM1.24 antibody.

### 6) Immunoprecipitation

After washing the cells twice in PBS (-), they were crushed by ultrasonication in the cell lysate buffer method (50 mM sodium acetate, 150 mM NaCl, 0.5% sodium deoxycholate, 1% Nonidet P-40, 0.1 mg/ml phenylmethylsulphonyl fluoride, protease inhibitor cocktail [Boehringer Mannheim]) to obtain a soluble fraction. The soluble fraction was added to anti-HM1.24 antibody-conjugated Sepharose 4B beads. After centrifugation, the precipitate was isolated on SDS-PAGE (12% gel), which was transferred to a PVDF membrane. The PVDF membrane was reacted with anti-HM1.24 antibody, and then with POD-anti-mouse IgG, and detected using the ECL kit (Amersham).

Each of myeloma cell lines KPMM2, RPMI8226, and U266 strongly expressed HM1.24 antigen, and immunoprecipitation of the cell lysates thereof with anti-HM1.24 antibody allowed the specific detection of proteins having a molecular weight of about 29-33 kDa (Figure 18). In a similar experiment for CHO cell lines (CHO/HM) in which P3.19 has been introduced, immunoprecipitants were confirmed in the CHO/HM cells as for the myeloma cell lines (Figure 18, lane 4). Such immunoprecipitants could not be confirmed in the control cells (CHO/NEO) in which the expression vector pCOS1 has only been introduced (Figure 18, lane 5).

P3.19 encodes a protein having an estimated molecular weight of 19.8 kDa comprising 180 amino acids (Figure 14). Thus, it suggested that the presence of substances having different molecular weights observed by immunoprecipitation was due to differences in the modification of N-type sugar chains. In fact, the immunoprecipitant has been confirmed to bind to several lectins.

### Explanation of sequence listing

SEQ ID NO: 1 shows the amino acid sequence and the nucleotide sequence of the extracellular domain of soluble HM1.24 antigen protein.
SEQ ID NO: 2 shows the amino acid sequence and the nucleotide sequence of a fusion protein comprising the leader sequence, the FLAG peptide and soluble HM1.24 antigen protein. A sequence comprising Met at position 1 to His at position 18 represents the leader sequence. A sequence comprising Asp at position 20 to Lys at position 27 represents the FLAG peptide. A sequence comprising Gly at position 28 to Thr at position 29 is a linker.
SEQ ID NO: 3 shows the amino acid sequence and the nucleotide sequence of a fusion protein comprising the HA peptide and the soluble HM1.24 antigen protein. A sequence comprising Tyr at position 1 to Ala at position 9 represents the HA peptide. A sequence comprising Gly at position 28 to Thr at position 29 is a linker.
SEQ ID NO: 4 shows the amino acid sequence and the nucleotide sequence of a fusion protein comprising the HA peptide and the C-terminal-deleted soluble HM1.24 antigen protein. A sequence comprising Tyr at position 1 to Ala at position 9 represents the HA peptide. A sequence comprising Gly at position 28 to Thr at position 29 is a linker.
SEQ ID NO: 5 shows the nucleotide sequence of the determined CGM/HA and the amino acid sequence of the HA peptide. A sequence comprising Tyr at position 1 to Ala at position 9 represents the HA peptide.
SEQ ID NO: 6 shows the amino acid sequence and the nucleotide sequence of the determined CGM/HA-HM164. A sequence comprising Met at position 1 to Cys at position 20 represents a leader sequence. A sequence comprising Gly at position 3 to Thr at position 32 is a linker. A sequence comprising Asn at position 33 to Ala at position 151 is the C-terminal-deleted soluble HM1.24 antigen protein.
SEQ ID NO: 7 shows the nucleotide sequence of primer CMV/L.
SEQ ID NO: 8 shows the nucleotide sequence of primer BGH-1.
SEQ ID NO: 9 shows the nucleotide sequence of primer SOl-1.
SEQ ID NO: 10 shows the nucleotide sequence of primer Sol-2.
SEQ ID NO: 11 shows the nucleotide sequence of primer Sol-3.
SEQ ID NO: 12 shows the nucleotide sequence of one of the synthetic DNA pair comprising a leader sequence and the FLAG peptide sequence.
SEQ ID NO: 13 shows the nucleotide sequence of the other of the synthetic DNA pair comprising a leader sequence and the FLAG peptide sequence.
SEQ ID NO: 14 shows the nucleotide sequence of one of the synthetic DNA pair having the HA peptide sequence.
SEQ ID NO: 15 shows the nucleotide sequence of the other of the synthetic DNA pair having the HA peptide sequence.
SEQ ID NO: 16 shows the amino acid sequence and the nucleotide sequence of human HM1.24 antigen protein expressed on the cell membrane.
SEQ ID NO: 17 shows the amino acid sequence and the nucleotide sequence of the a version of the L chain V region of humanized anti-HM1.24 antibody.
SEQ ID NO: 18 shows the amino acid sequence and the nucleotide sequence of the r version of the H chain V region of humanized anti-HM1.24 antibody.
SEQ ID NO: 19 shows the amino acid sequence and the nucleotide sequence of the s version of the H chain V region of humanized anti-HM1.24 antibody.

### Industrial Applicability

According to the immunochemical assay of the present invention, it is possible to detect or determine soluble HM1.24 antigen protein or anti-HM1.24 antibody having a concentration of up to about 500 pg/ml. The soluble HM1.24 antigen protein or anti-HM1.24 antibody that could so far be detected or determined only up to 10 ng/ml by the Cell Elisa can now be determined according to the present invention both sensitively and rapidly, and a large number of samples can be determined.

The soluble HM1.24 antigen protein of the present invention or DNA encoding it is useful for the determination of anti-HM1.24 antibody or soluble HM1.24 antigen protein.

### Reference to the microorganisms deposited under the Patent Cooperation Treaty, Rule 13-2, and the name of the

### Depository Institute

### Depository Institute

### Name: the National Institute of Bioscience and Human Technology, Agency of Industrial Science and Technology

### Address: 1-3, Higashi 1-chome, Tsukuba-shi, Ibaraki pref., Japan

### Organism (1)

Name: Escherichia coli DH5α (pRS38-pUC19)
Accession number: FERM BP-4434
Date deposited: October 5, 1993

### Organism (2)

Name: Mouse-mouse hybridoma HM1.24
Accession number: FERM BP-5233
Date deposited: September 14, 1995

### Organism (3)

Name: Escherichia coli DH5α (pUC19-1.24L-gκ)
Accession number: FERM BP-5646
Date deposited: August 29, 1996

### Organism (4)

Name: Escherichia coli DH5α (pUC19-1.24H-gγ1)
Accession number: FERM BP-5644
Date deposited: August 29, 1996

### Organism (5)

Name: Escherichia coli DH5α (pUC19-RVLa-AHN-gκ)
Accession number: FERN BP-5645
Date deposited: August 29, 1996

### Organism (6)

Name: Escherichia coli DH5α (pUC19-RVHr-AHM-gγ1)
Accession number: FERM BP-5643
Date deposited: August 29, 1996

### Organism (7)

Name: Escherichia coli DH5α (pUC19-RVHs-AHM-gγ1)
Accession number: FERM BP-6127
Date deposited: September 29, 1997

## Claims

1. An immunochemical assay for anti-HM1.24 antibody, said method comprising the steps of reacting soluble HM1.24 antigen protein and anti-HM1.24 antibody contained in a test sample, and then detecting or determining the anti-HM1.24 antibody bound to the soluble HM1.24 antigen protein.

2. The immunochemical assay according to claim 1 wherein said soluble HM1.24 antigen protein is bound to a support.

3. An immunochemical assay for soluble HM1.24 antigen protein, said method comprising the steps of reacting anti-HM1.24 antibody and soluble HM1.24 antigen protein contained in a test sample, and then detecting or determining the soluble HM1.24 antigen protein bound to the anti-HM1.24 antibody.

4. The immunochemical assay according to claim 3 wherein said anti-HM1.24 antibody is bound to a support.

5. The immunochemical assay according to any of claims 1 to 4 wherein said soluble HM1.24 antigen protein is fused to another peptide or polypeptide.

6. The immunochemical assay according to claim 2 or 4 wherein said support is beads or a plate.

7. The immunochemical assay according to any of claims 1 to 6 wherein the anti-HM1.24 antibody bound to the soluble HM1.24 antigen protein or the soluble HM1.24 antigen protein bound to the anti-HM1.24 antibody is detected or determined using a primary antibody against the anti-HM1.24 antibody or a primary antibody against the soluble HM1.24 antigen protein.

8. The immunochemical assay according to any of claims 1 to 7 wherein the anti-HM1.24 antibody bound to the soluble HM1.24 antigen protein or the soluble HM1.24 antigen protein bound to the anti-HM1.24 antibody is detected or determined using a primary antibody against the anti-HM1.24 antibody or a primary antibody against the soluble HM1.24 antigen protein, and a second antibody against said primary antibody.

9. The immunochemical assay according to any of claims 1 to 8 wherein the primary antibody or the second antibody is labeled with a radioisotope, an enzyme, biotin/avidin or a fluorogenic substance.

10. The soluble HM1.24 antigen protein having the amino acid sequence as set forth in SEQ ID NO: 1.

11. A fusion protein of the soluble HM1.24 antigen protein according to claim 10 and another peptide or polypeptide.

12. DNA encoding the soluble HM1.24 antigen protein or the fusion protein of the soluble HM1.24 antigen protein and another peptide or polypeptide according to claim 10 or 11.
